# EUROPEAN PATENT APPLICATION

(11) **EP 2 241 561 A1**
(43) Date of publication of application: **20.10.2010**
(21) Application number: 09382051.2
(22) Date of filing: 16.04.2009
(51) Int. Cl.: C07D 309/30, A61K 31/366, A61P 3/00, A61P 9/00, A61P 25/08, A61P 25/28, A61P 31/12

(54) **Neuroprotective, hypocholesterolemic and antiepileptic compound**

(71) Applicant: Neuron Biopharma, S.A., 18100 Armilla - Granada (ES)
(72) Inventor: Burgos Muñoz, Javier Santos, E-18100, Armilla - Granada (ES); Adrio Fondevila, José Luis, E-18100, Armilla - Granada (ES); Ramos Martín, Maria del Carmen, E-18100, Armilla - Granada (ES); Sierra Ávila, Saleta, E-18100, Armilla - Granada (ES); Alfaro Sánchez, Juan María, E-18100, Armilla - Granada (ES); Ramírez Moreno, Carlos, E-18100, Armilla - Granada (ES); Campoy García, Sonia, E-18100, Armilla - Granada (ES); Velasco Alvarez, Javier, E-18100, Armilla - Granada (ES); Rumbero Sánchez, Ángel, E-18100, Armilla - Granada (ES)
(74) Representative: ABG Patentes, S.L.

(57) **Abstract**

The present invention describes a compound of formula (I) 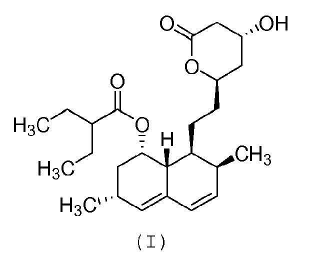 its hydroxy acid form, the pharmaceutically acceptable salts of said hydroxy acid and pharmaceutically acceptable prodrugs and solvates of the compound and of its hydroxy acid form and, in particular, said compound, its hydroxy acid form, salts, etc. for its use in the prevention of: neurodegenerative diseases, cognitive deterioration, diseases associated with undesired oxidation, age-associated pathological processes and progeria, epilepsy, epileptic seizures and convulsions, cardiovascular diseases such as atherosclerosis, atrial fibrillation, dyslipemia, hypercholesterolemia, hyperlipidemia, and hypertriglyceridemia, or fungal or viral infections.

## Description

### Field of the Invention

The present invention relates to the prevention and/or the treatment of neurodegenerative diseases or of diseases associated with an unwanted oxidation or of age-associated pathological processes, as well as to the prevention and/or the treatment of epilepsy, of epileptic seizures or of convulsions, to the decrease of LDL cholesterol levels and to the inhibition of the enzyme 3-hydroxy-3-methylglutaryl coenzyme A reductase for the prevention of dyslipemia and of cardiovascular diseases.

### Background of the Invention

The high incidence of neurodegenerative diseases and of age-associated diseases is a problem of the first order worldwide. It is therefore necessary to search for neuroprotective compounds preventing or palliating said diseases. Of all of them, Alzheimer's disease (AD) is the most prevalent, it being estimated that 81 million people will suffer from this disease in 2040 (Blennow et al., Lancet 2006; 368: 387-403). It is estimated that half a million people are currently suffering from AD in Spain alone. The costs associated to this disease are proportionally high, and it is calculated that the total cost derived from caring for Alzheimer's patients is 81,000 and 22,000 million € in the United States and in the United Kingdom, respectively. Currently there are no effective drugs which prevent or impede this disease, therefore it is necessary to search for and validate novel neuroprotective compounds which prevent neuronal damage.

Different strategies are currently being followed for obtaining novel compounds, since it has been seen that the current drugs offer little benefits to the patients. These drugs temporarily delay (one year, at best) some symptoms of the illness but do not prevent their evolution. The current therapeutic options are based on the inhibition of acetylcholinesterase with drugs such as donepezil, galantamine or rivastigmine, or on the capacity of memantine in antagonizing a glutamate receptor, NMDA (N-methyl-D-aspartic acid).

Due to the low success of these drugs new lines of research have opened up and among them, research on inhibitors of the 3-hydroxy-3-methylglutaryl-coenzyme A reductase (HMGR) enzyme (known as statins) as therapeutic agents stands out in recent years. HMGR is the enzyme which catalyzes the limiting step in cholesterol biosynthesis; therefore its inhibition by statins is a usual therapeutic strategy to reduce the high cholesterol levels associated to the low density lipoproteins (LDL). These drugs reduce the risk of myocardial infarction and coronary death, and are considered safe. Moreover, hypercholesterolemia (defined as a high blood cholesterol level) is the main risk factor for ischemic cardiovascular disease, such as atherosclerosis.

Several genetic and environmental factors affecting cholesterol metabolism are associated with AD. For example, the apolipoprotein E ε4 (apoE4) isoform is a risk factor for AD and is linked to an increase in cholesterol levels. Atherosclerosis, which has hypercholesterolemia as the main risk factor, also seems to be associated to AD. Furthermore, epidemiological studies indicate that high serum cholesterol levels increase the risk of AD, and it has been proposed that homeostatic regulation of cholesterol metabolism can be altered in Alzheimer's. On the other hand, a significant reduction of the risk of Alzheimer's in patients treated with statins has been described. All these studies jointly suggest that the reduction of cholesterol levels can inhibit the pathogenesis of Alzheimer's disease (Cole & Vassar; Neurobiol Dis 2006; 22[2]:209-22).

Cholesterol is transported through blood by means of different types of lipoproteins, in which the major cholesterol carriers are low density lipoproteins (LDL) and high density lipoproteins (HDL). LDLs are lipoproteins specialized in transporting cholesterol and triglycerides from the liver to peripheral tissues, in which they are captured by the cells through the LDL receptors (LDL-R) in cell membrane. LDLs also regulate cholesterol synthesis, and high LDL cholesterol levels have been associated to the risk of suffering from cardiovascular diseases (CVD). In turn, HDLs are lipoproteins which transport cholesterol from the different tissues to the liver. Due to the fact that HDLs can remove cholesterol from arteries and transport it back to the liver for its excretion, they are given a protective role against cardiovascular diseases. HMGR inhibitors are the most successful hypolipidemic agents in history, being capable of reducing total cholesterol levels based on decreasing LDL cholesterol levels without altering HDL cholesterol levels.

New properties of the statins have recently been described, especially at the level of brain damage caused by trauma or in dementias, new antioxidant and anti-inflammatory activities being proposed (Pahan, Cell Mol Life Sci. 2006; 63[10]:1165-78), and certain statins (e.g., simvastatin) have been demonstrated to intensify the learning and memory capacity in mice (Ling et al., Ann Neurol. 2006; 60[6]:729-39) or protect them against convulsive seizures associated to epileptic phenomena (Lee et al., Neurosci Lett. 2008; 440: 260-4). Moreover, the statins have also demonstrated their effectiveness in phase II clinical trials which suggest positive results against the treatment of cerebral vasospasm (Fandino et al., Neurocirugia. 2007; 18: 16-27), as well as against neuronal death induced by ischemic damage in the retina (Honjo et al., Arch. Ophthalmol. 2002; 120: 1707-13). Nevertheless, it is currently being discussed whether the neuroprotective effects of the different commercial statins (e.g., atorvastatin, lovastatin, simvastatin, etc.) are due to a direct effect on the lipid metabolism, or whether in contrast they are a result of alternative routes.

Patent application WO 99/11258 describes a compound with a structure similar to the one of the present invention. Nevertheless, this document does not specify the configuration of the different chiral centers present in the compound.

### Summary of the Invention

Although there is literature on the potential neuroprotective effect of statins, the authors of this invention have found that a derivative of a non-commercial monacolin J, specifically (1S,2S,6R,8S,8aR)-1,2,6,7,8,8a-hexahydro-3,7-dimethyl-8-[2-[(2R,4R)-tetrahydro-4-hydroxy-6-oxo-2H-pyran-2-yl]ethyl]-1-naphthalenyl-2-ethyl-butyrate (also sometimes identified as NST0037 in this patent application) is an agent with a surprisingly neuroprotective potential, in addition to having a high capacity of reducing blood lipid (cholesterol and triglycerides) levels, very effectively inhibiting HMGR, and protecting against epilepsy, epileptic seizures and convulsions. Furthermore, and surprisingly, this compound is safer than commercial statins, showing toxicity levels under the levels of the statin showing the highest level of biosafety, simvastatin. Additionally, it refers to a compound which is less expensive to synthesize due to the low cost of the side chain to be added to the monacolin J molecule.

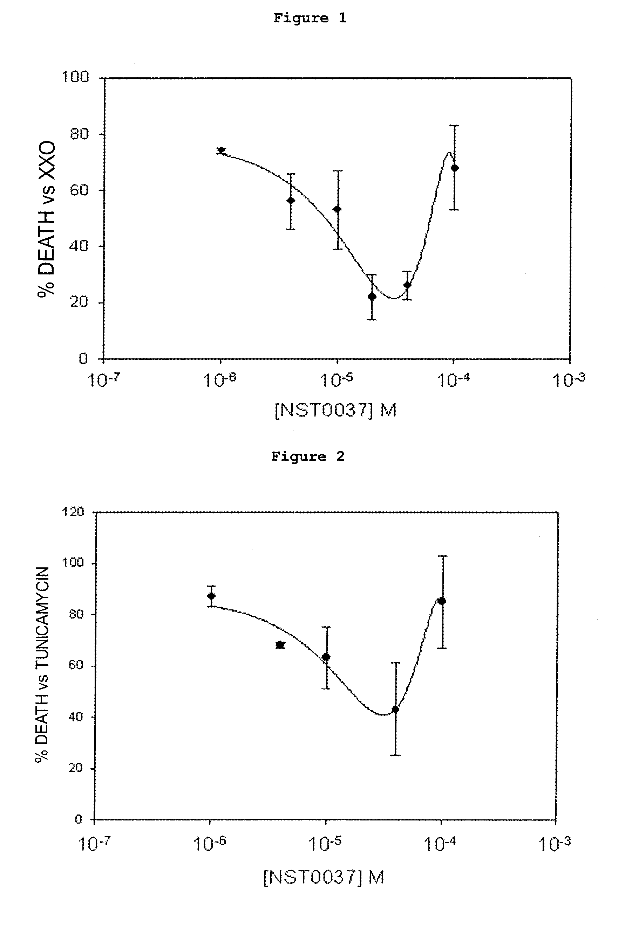

The neuroprotective activity of said compound has been clearly shown against different aggressions which cause neuronal death in human cell lines of cholinergic origin by means of different types of aggressions which cause oxidative stress, reticular stress or apoptosis (Example 2, Figures 1 to 4). Said example clearly shows the potential use of said compound in the prevention and/or treatment of neuronal death associated to neurodegenerative diseases (e.g., Alzheimer's, Parkinson's, multiple sclerosis, amyotrophic lateral sclerosis, *status* epilepticus, Huntington's, etc.) or of diseases associated with undesired oxidation or of age-associated pathological processes.

The neuroprotective activity of said compound has been confirmed in a mouse model of Alzheimer's disease, in which this compound exerts a neuroprotective effect against neuronal death in the hippocampus caused by an excitotoxic substance (Example 3, Figure 5). Furthermore, it has been found that said substance restores temporal and spatial memory in mice with neurodegeneration (Example 3, Figures 6 and 7), in addition to preventing death of animals caused by the administration of an excitotoxic substance (Example 3, Figure 8). Said examples clearly show the potential use of this compound in the prevention and/or treatment of neuronal death and of the cognitive deficit associated to neurodegenerative diseases (e.g., Alzheimer's, Parkinson's, multiple sclerosis, amyotrophic lateral sclerosis, *status epilepticus,* Huntington's, etc.) or of diseases associated with undesired oxidation or of age-associated pathological processes.

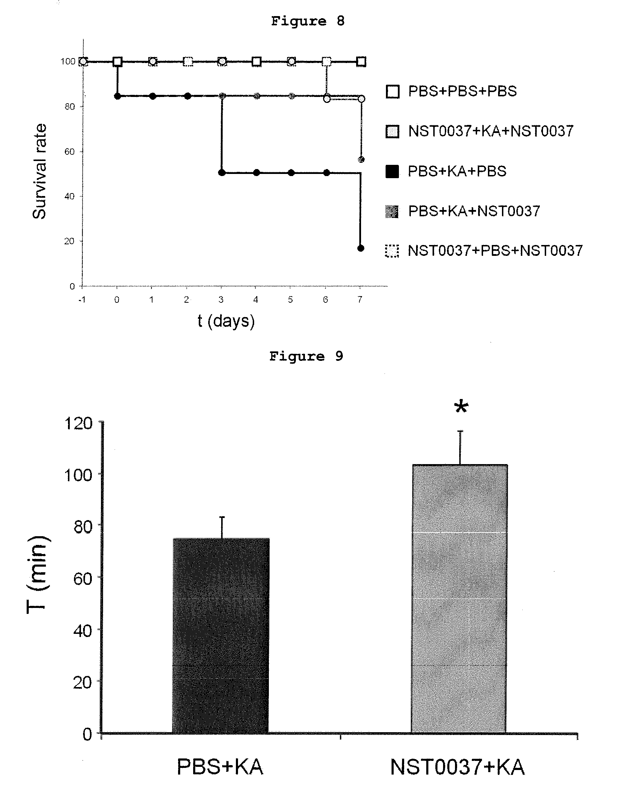

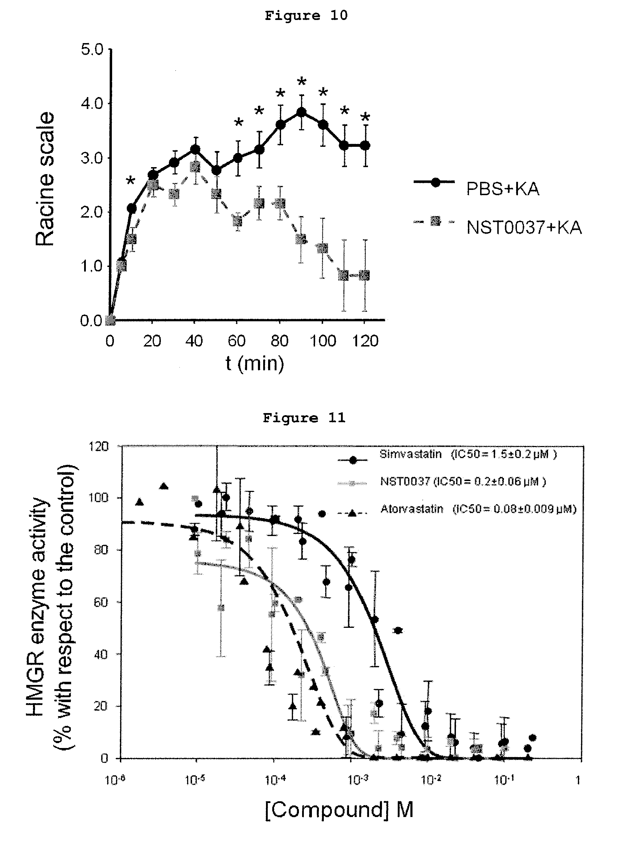

The antiepileptic and anticonvulsant activity of said compound has been clearly shown by means of the determination of the protection against epileptic seizures and convulsions in an epilepsy model in mice (Example 4, Figures 9 and 10). Said example clearly shows the potential use of this compound in the prevention and/or treatment of epilepsy and convulsive seizures or convulsions.

The hypolipidemic activity of said compound has been clearly shown by means of the determination of the HMGR inhibition in comparison to two commercial statins, simvastatin and atorvastatin (Example 5, Figure 11).

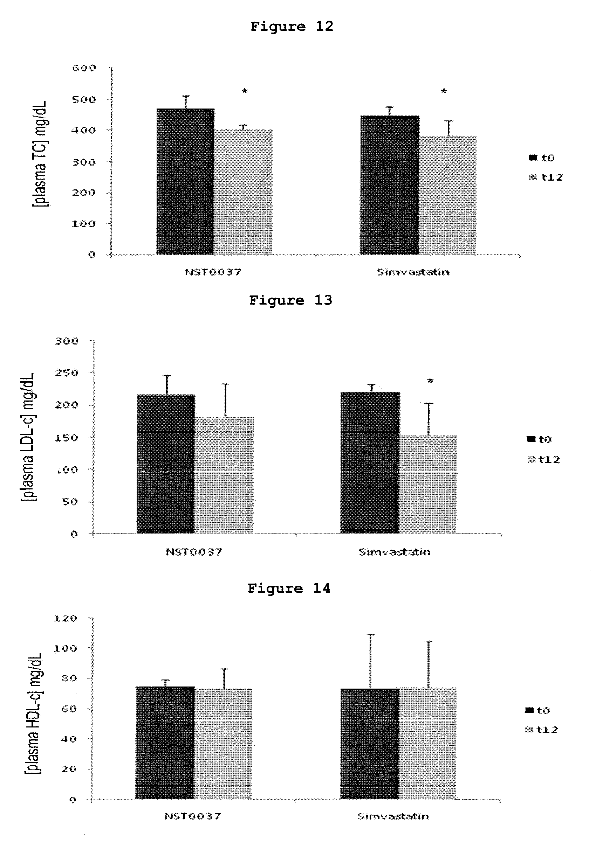

The hypolipidemic capacity of said compound has additionally been demonstrated in an endogenous hyperlipidemia model in mice, an effect similar to simvastatin in reducing total plasma cholesterol levels, LDL cholesterol levels, VLDL cholesterol levels and esterified cholesterol fraction levels being observed. In contrast, and like with simvastatin, it alters neither HDL cholesterol levels nor free cholesterol fraction levels, giving it a protective role against cardiovascular diseases (CVD) (Example 5, Figures 12 to 17). Said examples clearly show the potential use of said compound in the prevention and/or treatment of hypercholesterolemia associated to cardiovascular diseases (e.g., myocardial infarction, atherosclerosis, congenital cardiopathy, acquired cardiopathy, ischemic cardiopathy, hypertensive cardiopathy, valvulopathies, cardiomyopathies, blood disorders, etc.).

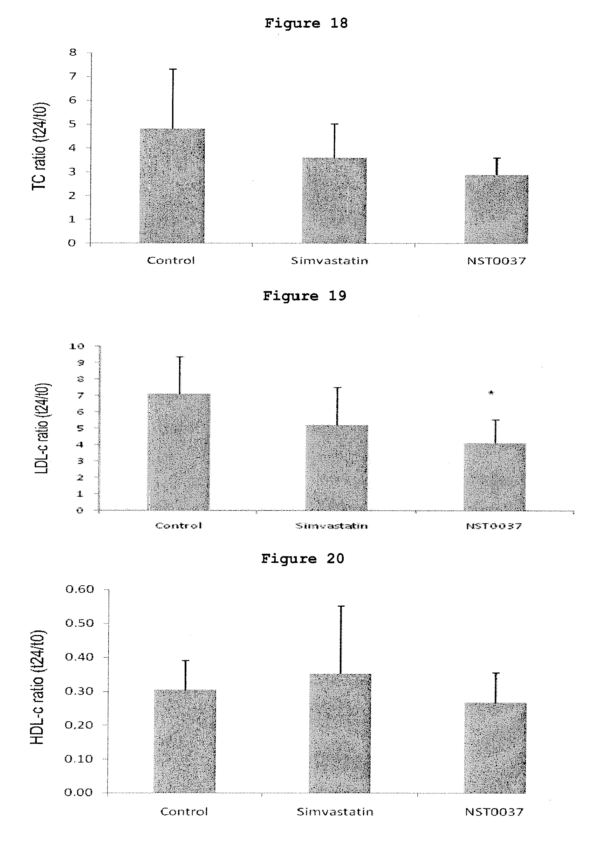

The hypolipidemic activity of said compound has been confirmed in an induced hyperlipidemia model in mice (Example 6, Figures 18 to 23), an effect greater than simvastatin in reducing the total plasma cholesterol levels, LDL cholesterol levels and free and esterified cholesterol fraction levels being observed. In contrast, this compound alters neither HDL cholesterol levels nor VLDL cholesterol levels, giving it a protective role against cardiovascular diseases (CVD). Said examples clearly show the potential use of said compound in the prevention and/or treatment of hypercholesterolemia associated to cardiovascular diseases (e.g., myocardial infarction, atherosclerosis, congenital cardiopathy, acquired cardiopathy, ischemic cardiopathy, hypertensive cardiopathy, valvulopathies, cardiomyopathies, blood disorders, etc.).

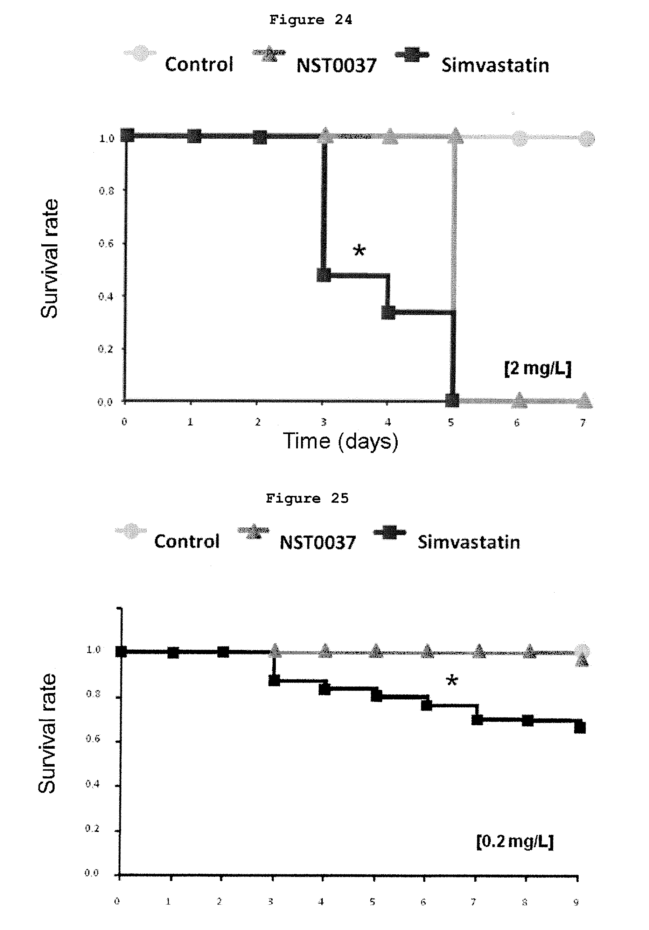

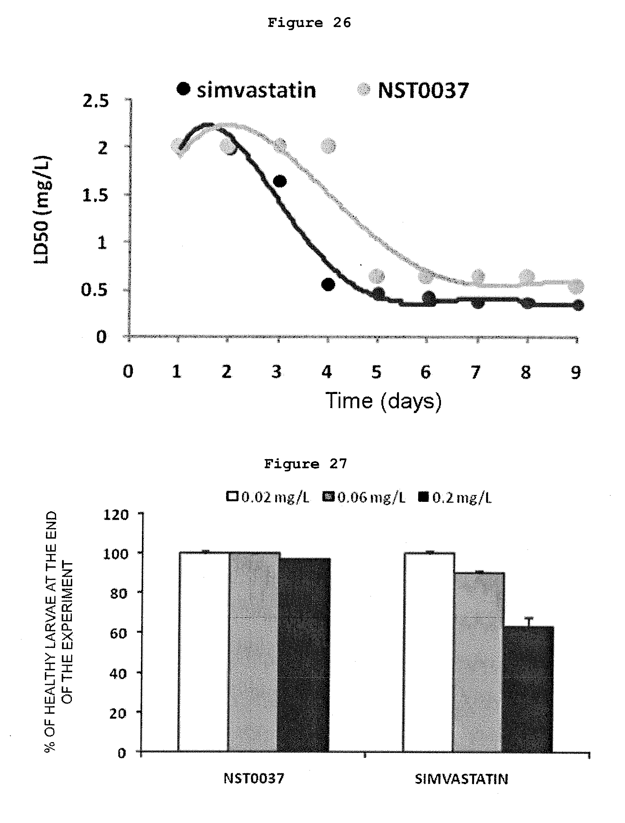

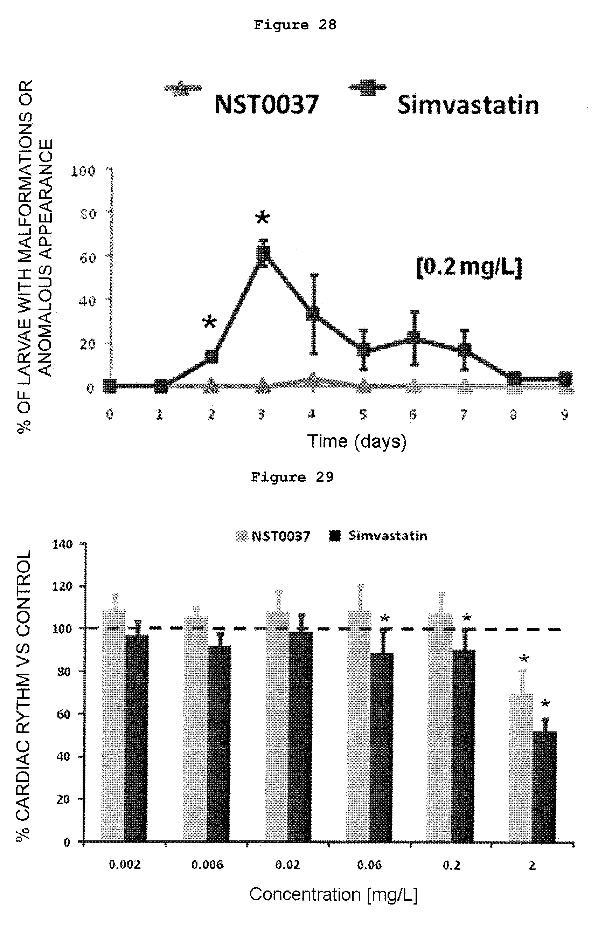

The biosafety of said compound has been clearly shown by means of the evaluation of its toxicity in a zebrafish embryo model in comparison with a commercial statin, simvastatin, observing that it is less toxic than simvastatin at different concentrations since a lower mortality occurs (Example 7, Figures 24 and 25). Additionally, it has been demonstrated that the lethal dose 50 (LD50) is higher in the case of compound NST0037 than in the case of simvastatin at all the evaluated time points, indicating a higher biosafety of said compound (Example 7, Figures 26). Additionally, it has been demonstrated that this compound causes a higher percentage of healthy larvae at the end of the experiment, as well as a lower percentage of larvae with malformations or anomalous appearance (Example 7, Figures 27 and 28). Additionally, this compound does not cause a significant variation of the percentage of heartbeats at high concentrations, unlike simvastatin which causes a significant reduction of the cardiac rhythm at high concentrations (Example 7, Figures 29).

Therefore, one aspect of the present invention relates to a compound of formula (I) [also identified on occasions in this patent application as NST0037]: 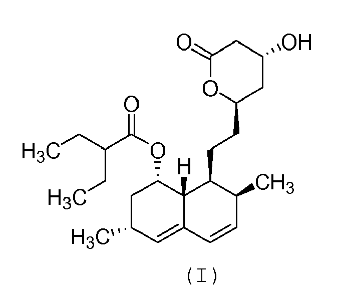 its hydroxy acid form, the pharmaceutically acceptable salts of said hydroxy acid and pharmaceutically acceptable prodrugs and solvates of the compound and of its hydroxy acid form.

Another aspect of the present invention is a pharmaceutical composition comprising a compound of formula (I) and/or its hydroxy acid form and/or a pharmaceutically acceptable salt of said hydroxy acid and/or a pharmaceutically acceptable prodrug or solvate of the compound or of its hydroxy acid form, and at least one pharmaceutically acceptable adjuvant, carrier and/or vehicle.

Another aspect of the present invention relates to a compound of formula (I), its hydroxy acid form or a pharmaceutically acceptable salt of said hydroxy acid and/or a pharmaceutically acceptable prodrug or solvate of the compound or of its hydroxy acid form for its use as medicament.

According to another aspect, the present invention relates to a compound of formula (I), its hydroxy acid form or a pharmaceutically acceptable salt of said hydroxy acid and/or a pharmaceutically acceptable prodrug or solvate of the compound or of its hydroxy acid form for its use as a neuroprotective agent, in particular in the prevention and/or the treatment of:
a. neurodegenerative diseases (e.g., Alzheimer's, Parkinson's, multiple sclerosis, amyotrophic lateral sclerosis, *status* epilepticus, Huntington's, etc.), more specifically, as a neuroprotective agent against apoptotic processes, oxidative stress or endoplasmic reticulum stress associated to said chronic neurodegenerative diseases,
b. cognitive deterioration,
c. diseases associated with undesired oxidation,
d. age-associated pathological processes and progeria,
e. epilepsy, epileptic seizures and convulsions,
f. cardiovascular diseases such as atherosclerosis, atrial fibrillation, dyslipemia, hypercholesterolemia, hyperlipidemia, and hypertriglyceridemia, or
g. fungal or viral infections

One aspect of the present invention relates to the use of a compound of formula (I), of its hydroxy acid form, of a pharmaceutically acceptable salt of said hydroxy acid and/or of a pharmaceutically acceptable prodrug or solvate of the compound or of its hydroxy acid form in the manufacture of a medicament. According to a particular embodiment, the medicament is for being used as a neuroprotective agent, in particular in the prevention and/or the treatment of:
a. neurodegenerative diseases (e.g., Alzheimer's, Parkinson's, multiple sclerosis, amyotrophic lateral sclerosis, *status* epilepticus, Huntington's, etc.), more specifically, as a neuroprotective against apoptotic processes, oxidative stress or endoplasmic reticulum stress associated to said chronic neurodegenerative diseases,
b. cognitive deterioration,
c. diseases associated with undesired oxidation,
d. age-associated pathological processes and progeria,
e. epilepsy, epileptic seizures and convulsions,
f. cardiovascular diseases such as atherosclerosis, atrial fibrillation, dyslipemia, hypercholesterolemia, hyperlipidemia, and hypertriglyceridemia, or
g. fungal or viral infections

Another aspect of the present invention is a compound of formula (I), its hydroxy acid form or a pharmaceutically acceptable salt of said hydroxy acid and/or a pharmaceutically acceptable prodrug or solvate of the compound or of its hydroxy acid form for its use in increasing seladin-1/DHCR24 gene expression.

Another aspect of the present invention is a compound of formula (I), its hydroxy acid form or a pharmaceutically acceptable salt of said hydroxy acid and/or a pharmaceutically acceptable prodrug or solvate of the compound or of its hydroxy acid form for its use in the prevention and/or treatment of diseases related to the seladin-1/DHCR24 gene.

Another aspect of the present invention relates to the use of a compound of formula (I), of its hydroxy acid form, of a pharmaceutically acceptable salt of said hydroxy acid and/or of a pharmaceutically acceptable prodrug or solvate of the compound or of its hydroxy acid form in the manufacture of a medicament characterized by increasing seladin-1/DHCR24 gene expression.

In another aspect, the invention relates to a method for the prevention and/or treatment of neurodegenerative diseases, cognitive deterioration, diseases associated with undesired oxidation, age-associated pathological processes and progeria, epilepsy, epileptic seizures and convulsions, cardiovascular diseases such as atherosclerosis, atrial fibrillation, dyslipemia, hypercholesterolemia, hyperlipidemia and hypertriglyceridemia, or fungal or viral infections in a subject in need of treatment, comprising administering to said subject a therapeutically effective amount of a compound of formula (I), its hydroxy acid form or a pharmaceutically acceptable salt of said hydroxy acid and/or a pharmaceutically acceptable prodrug or solvate of the compound or of its hydroxy acid form.

### Brief Description of the Figures

Figure 1 is an XY scatter chart depicting the protective effect of compound NST0037 against death caused by xanthine/xanthine oxidase (XXO). The figure shows the percentage of cell death (taking as 100% the cell death caused by XXO) of the cultures treated with 10 µM xanthine (X) 60 mU/mL xanthine oxidase (XO) and NST0037 at different concentrations, representing the meanj_SD of 3 independent experiments in triplicate. *Significant difference with respect to the treatments with XXO alone, according to the Student's t test (p<0.05).
Figure 2 is an XY scatter chart depicting the protective effect of compound NST0037 against death caused by tunicamycin (Tm). The figure shows the percentage of cell death (taking as 100% the cell death caused by Tm) of the cultures treated with 24 µM Tm and NST0037 at different concentrations, representing the mean±SD of 3 independent experiments in triplicate. *Significant difference with respect to the treatments with Tm alone, according to the Student's t test (p<0.05).

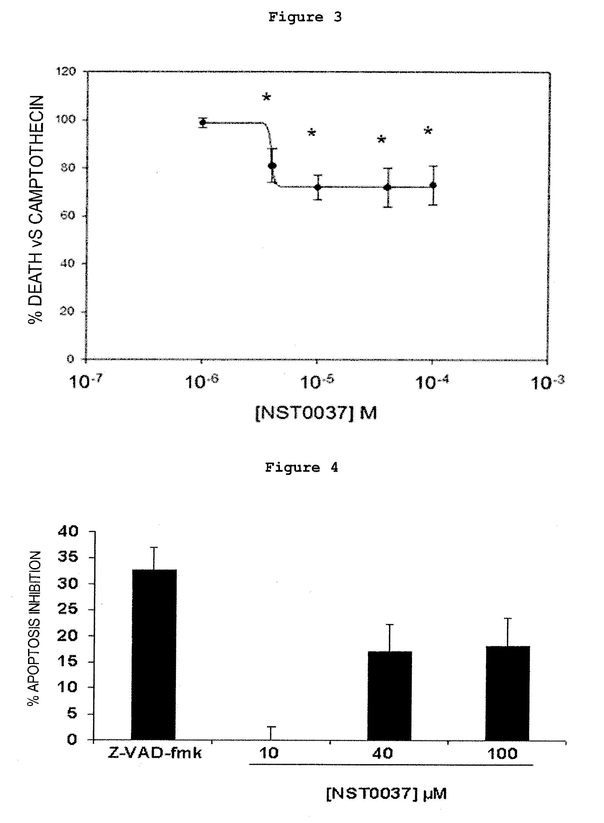

Figure 3 is an XY scatter chart depicting the protective effect of compound NST0037 against death caused by camptothecin (Camp). The figure shows the percentage of cell death (taking as 100% the cell death caused by Camp) of the cultures treated with 20 nM Camp and NST0037 at different concentrations, representing the mean±SD of 3 independent experiments in triplicate. *Significant difference with respect to the treatments with Camp alone, according to the Student's t test (p<0.05).
Figure 4 is a bar graph depicting the protective effect of compound NST0037 against apoptosis caused by camptothecin (Camp) determined by flow cytometry. The figure shows the percentage of inhibition of apoptosis caused by 50 µM Camp and NST0037 at 10, 40 and 100 µM in comparison with the inhibition control, Z-VAD-fmk at 50 µM, representing the mean±SD of 3 independent experiments. *Significant difference with respect to the treatment with Camp alone, according to the Student's t test (p<0.05).

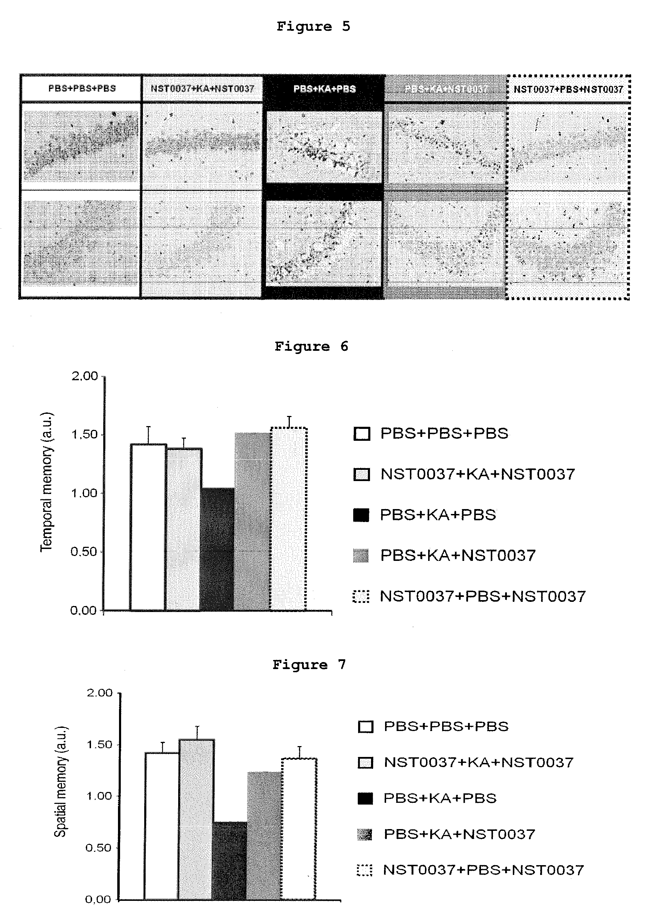

Figure 5 is a micrograph composition showing the hippocampal CA1 and CA2 regions of mice in which the samples have been stained with hematoxylin and eosin. The figure depicts the histopathological analysis of the cell structure of these regions according to the pretreatment (24 and 0.5 hours before the inoculation of the excitotoxic substance [kainic acid or kainate or KA]), the inoculation of the excitotoxic substance (0 days post-inoculation [d.p.i.]) and the subsequent treatment (up to 7 d.p.i.).
Figure 6 is a bar graph in which the state of the temporal memory is analyzed according to the protocol by Dere et al.
   (Dere, E., Huston, J. P. & De Souza Silva, M. A. Neurobiol Learn Mem 2005; 84: 214-21). The bars represent the means±SEM of the temporal memory expressed in arbitrary units (y-axis), according to the pretreatment (24 and 0.5 hours before the inoculation of the excitotoxic substance [kainic acid or kainate or KA]), the inoculation of the excitotoxic substance (0 days post-inoculation [d.p.i.]) and the subsequent treatment (up to 7 d.p.i.).
Figure 7 is a bar graph in which the state of the spatial memory is analyzed according to the protocol by Dere et al. (Dere, E., Huston, J. P. & De Souza Silva, M. A. Neurobiol Learn Mem 2005; 84: 214-21). The bars represent the means±SEM of the spatial memory expressed in arbitrary units (y-axis), according to the pretreatment (24 and 0.5 hours before the inoculation of the excitotoxic substance [kainic acid or kainate or KA]), the inoculation of the excitotoxic substance (0 days post-inoculation [d.p.i.]) and the subsequent treatment (up to 7 d.p.i.).
Figure 8 is a Kaplan-Meier graph depicting the survival rate of mice according to the pretreatment (24 and 0.5 hours before the inoculation of the excitotoxic substance [kainic acid or kainate or KA]), the inoculation of the excitotoxic substance (0 days post-inoculation [d.p.i.]) and the subsequent treatment (up to 7 d.p.i.).
Figure 9 is a bar graph wherein the mean±SEM of the time is represented in minutes after the inoculation of KA in which the first convulsion (y-axis) occurs according to the pretreatment received (x-axis). The group of pretreatment with PBS is represented in black and the group of treatment with NST0037 at 50 mg/Kg by weight is represented in gray.
Figure 10 is an XY scatter chart depicting the severity level of the *status epilepticus* observed according to Racine's scale (Racine, Electroencephalogr Clin Neurophysiol 1972; 32[3]:281-94) against the post-inoculation time of the epileptogenic substance (kainic acid or kainate or KA). The chart shows the evolution of the epileptogenic state of the animals according to the treatment received: PBS is represented with black circles and lines and the group of treatment with NST0037 at 50 mg/Kg by weight is represented with gray squares and lines.
Figure 11 is an XY scatter chart depicting the dose-response curves of compound NST0037 in comparison with two commercial statins (simvastatin and atorvastatin) on the HMGR enzyme activity *in vitro.* The chart shows the percentage of HMGR enzyme activity with respect to the control of the compounds at different doses, representing the mean±SD of at least four independent assays.
Figure 12 is a bar diagram depicting total plasma cholesterol levels of male ApoB100 mice 12 hours after being treated with 50 mg/kg of NST0037 or simvastatin, representing the mean±SD of each of the groups. *Significant difference with respect to time 0 hours, according to the Student's t test (p<0.05).
Figure 13 is a bar diagram depicting the plasma LDL cholesterol (LDL-c) levels of male ApoB100 mice 12 hours after being treated with 50 mg/kg of NST0037 or simvastatin, representing the mean±SD of each of the groups. *Significant difference with respect to time 0 hours, according to the Student's t test (p<0.05).
Figure 14 is a bar diagram depicting the plasma HDL cholesterol levels (HDL-c) of male ApoB100 mice 12 hours after being treated with 50 mg/kg of NST0037 or simvastatin, representing the mean±SD of each of the groups. *Significant difference with respect to time 0 hours, according to the Student's t test (p<0.05).

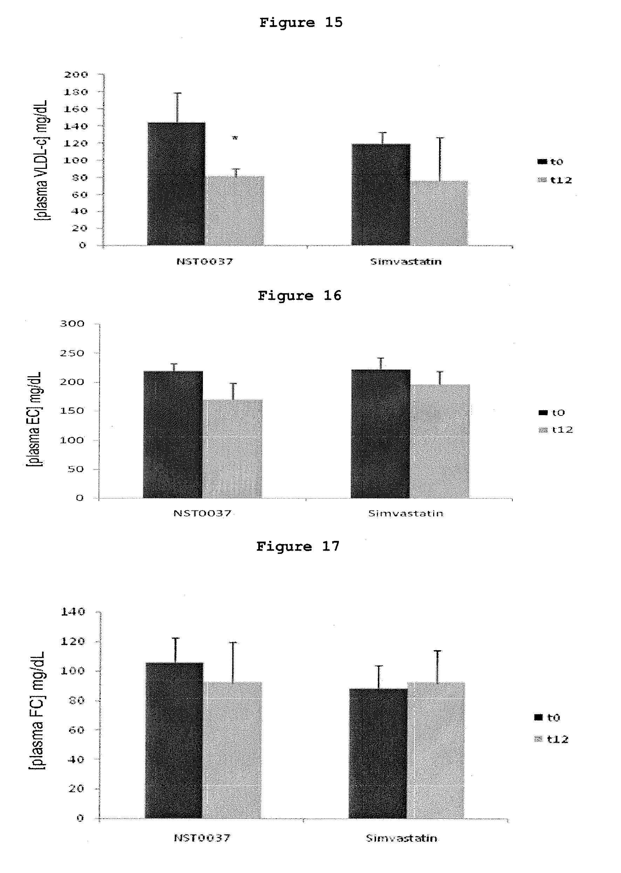

Figure 15 is a bar diagram depicting the plasma VLDL cholesterol levels (VLDL-c) of male ApoB100 mice 12 hours after being treated with 50 mg/kg of NST0037 or simvastatin, representing the mean±SD of each of the groups. *Significant difference with respect to time 0 hours according to the Student's t test (p<0.05).
Figure 16 is a bar diagram depicting the plasma esterified cholesterol (EC) levels of male ApoB100 mice 12 hours after being treated with 50 mg/kg of NST0037 or simvastatin, representing the mean±SD of each of the groups. *Significant difference with respect to time 0 hours according to the Student's t test (p<0.05).
Figure 17 is a bar diagram depicting the plasma free cholesterol (FC) levels of male ApoB100 mice 12 hours after being treated with 50 mg/kg of NST0037 or simvastatin, representing the mean±SD of each of the groups. *Significant difference with respect to time 0 hours according to the Student's t test (p<0.05).
Figure 18 is a bar diagram depicting the number of times the total cholesterol (TC) in male C57BL6 mice increases after 24 hours of being treated i.p. with 500 mg/kg of Triton 1339 (Tyloxapol) and 50 mg/kg of NST0037 or simvastatin, representing the mean±SD of each of the groups. *Significant difference with respect to the control group, according to the Student's t test (p<0.05).
Figure 19 is a bar diagram depicting the number of times the LDL cholesterol (LDL-c) in male C57BL6 mice increases after 24 hours of being treated i.p. with 500 mg/kg of Triton 1339 (Tyloxapol) and 50 mg/kg of NST0037 or simvastatin, representing the mean±SD of each of the groups. *Significant difference with respect to the control group, according to the Student's t test (p<0.05).
Figure 20 is a bar diagram depicting the number of times the HDL cholesterol (HDL-c) in male C57BL6 mice increases after 24 hours of being treated i.p. with 500 mg/kg of Triton 1339 (Tyloxapol) and 50 mg/kg of NST0037 or simvastatin, representing the mean±SD of each of the groups.

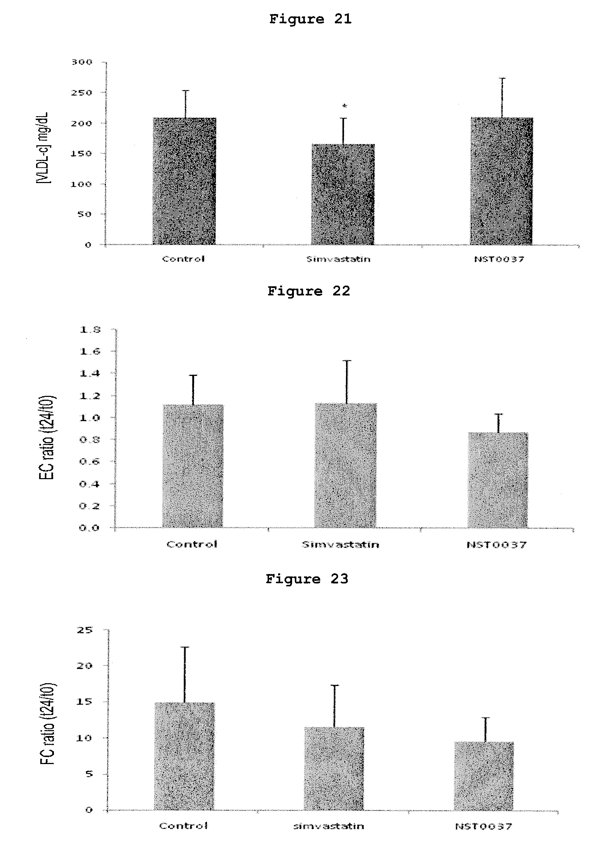

Figure 21 is a bar diagram depicting the VLDL cholesterol levels (VLDL-c) in male C57BL6 mice after 24 hours of being treated i.p. with 500 mg/kg of Triton 1339 (Tyloxapol) and 50 mg/kg of NST0037 or simvastatin, representing the mean±SD of each of the groups. *Significant difference with respect to the control group, according to the Student's t test (p<0.05).
Figure 22 is a bar diagram depicting the number of times the esterified cholesterol (EC) in male C57BL6 mice increases after 24 hours of being treated i.p. with 500 mg/kg of Triton 1339 (Tyloxapol) and 50 mg/kg of NST0037 or simvastatin, representing the mean±SD of each of the groups.
Figure 23 is a bar diagram depicting the number of times the free cholesterol (FC) in male C57BL6 mice increases after 24 hours of being treated i.p. with 500 mg/kg of Triton 1339 (Tyloxapol) and 50 mg/kg of NST0037 or simvastatin, representing the mean±SD of each of the groups.
Figure 24 is a Kaplan-Meier graph depicting the survival rate of the embryos-larvae according to the treatment received:
   control, NST0037 (at a dose of 2 mg/L) or simvastatin (at a dose of 2 mg/L). *Significant difference with respect to the control, according to the χ² test (p<0.01).
Figure 25 is a Kaplan-Meier graph depicting the survival rate of the embryos-larvae according to the treatment received:
   control, NST0037 (at a dose of 0.2 mg/L) or simvastatin (at a dose of 2 mg/L). *Significant difference with respect to the control, according to the χ² test (p<0.01).
Figure 26 is an XY scatter chart depicting the lethal dose 50 (LD50) of the two compounds (NST0037 or simvastatin) against the time of treatment.
Figure 27 is a bar graph depicting the percentage of healthy larvae which is obtained at the end of the experiment according to the treatment received (NST0037 or simvastatin) and the doses used (0.02, 0.06 or 0.2 mg/L), and wherein the means ± SD are represented.
Figure 28 is an XY scatter chart depicting the percentage of embryos-larvae with malformations or anomalous appearance according to the treatment received (NST0037 or simvastatin). *Significant difference between the two treatments, according to the Student's t test (p<0.05).
Figure 29 is a bar graph depicting the percentage of the cardiac rhythm of the embryos-larvae treated with increasing doses of the compounds NST0037 or simvastatin, at 72 hours posttreatment, representing the means ± SD. The horizontal dotted black line represents the mean value of the cardiac rhythm corresponding to the controls. *Significant difference of the treatments with respect to the control, according to the Student's t test (p<0.05).

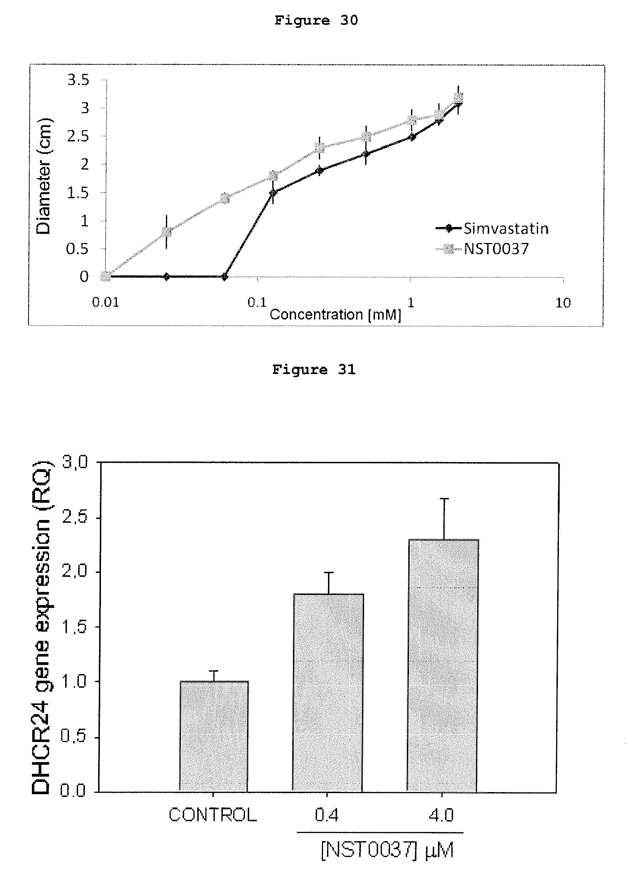

Figure 30 is a chart showing the antifungal activity of compound NST0037 and of simvastatin. The logarithm of the assayed concentrations (mM) is represented therein against the diameter of the inhibition halos (cm).
Figure 31 is a bar graph showing the increase of seladin-1/DHCR24 gene expression due to treatment with NST0037 in SK-N-MC cells. The relative quantification (RQ) of seladin-1/DHCR24 gene expression is shown, normalized by 18S, and referring to the value of the untreated cells (control), for the treatments for 24 h with NST0037 at 0.4 µM and 4 µM. The results of a representative assay are shown.

### Detailed Description of the Invention

### Definitions

To aid in understanding the invention object of this patent application, the meaning of some terms and expressions used in the context of the invention is explained below.

The term "neuroprotective agent", as it is used herein, relates to any substance capable of causing the attenuation or disappearance of the effects of neuronal degeneration or death by means of any mechanism known or to be known, for example, necrosis, apoptosis, autophagia, oxidative damage, excitotoxicity, endoplasmic reticulum damage, deposition of byproducts, loss of cell architecture, etc., or to the reduction or disappearance of the side effects thereof.

The term "statin", as it is used herein, relates to an inhibitor of the 3-hydroxy-3-methylglutaryl-coenzyme A reductase (HMGR) enzyme, which catalyzes the limiting step of cholesterol biosynthesis and includes any natural, synthetic or semisynthetic statin. Some statins can be in the closed form (lactone) or in the open form (hydroxy acid). Hydroxy acids (open form) can be prepared from the corresponding lactones by conventional hydrolysis, for example, with sodium hydroxide in methanol, sodium hydroxide in tetrahydrofuran-water and the like. In the open form (hydroxy acid), the statins react to form salts with pharmaceutically acceptable metal and amine cations formed from organic or inorganic bases. The pharmaceutically acceptable salts of the statins can differ from the corresponding free acids in some physical characteristics such as solubility and melting point, but they are considered equivalent to the free acid form for the purposes of this invention. The free open form (hydroxy acid) of the statins can be regenerated from the salt form, if desired, by contacting the salt with a diluted aqueous solution of an acid such as hydrochloric acid and the like. The closed form (lactone) of the statins can be regenerated by dissolving the open form (hydroxy acid) in an inert solvent such as, for example, toluene, benzene, ethyl acetate and the like, at temperatures comprised between approximately 0°C and approximately the boiling point of the solvent, typically (although not necessarily) with simultaneous separation of the resulting water and catalysis with strong acids, e.g., hydrochloric acid and the like. Likewise, the statins can exist in a solvated or non-solvated form and such forms are equivalent to the non-solvated form for the purposes of this invention.

The term "cardioprotective", as it is used herein, relates to any substance capable of causing the attenuation or disappearance of the underlying effects of cardiovascular diseases or cardiopathies or of cardiac damage by means of any mechanism known or to be known, for example, necrosis, apoptosis, ischemia, arrhythmia, deposition of byproducts, loss of cell architecture, etc., or to the reduction or disappearance of the side effects thereof.

The term "hypolipidemic", as it is used herein, relates to any pharmacologically active substance having the property of reducing blood lipid levels or lipid levels in other tissues. The importance of these substances is due to the fact that the excess of some types of lipids (cholesterol or triglycerides) or lipoproteins is one of the main risk factors for cardiovascular diseases.

The term "hypocholesterolemic", as it is used herein, relates to any pharmacologically active substance having the property of reducing blood cholesterol levels or cholesterol levels in other tissues.

The term "hypotriglyceridemic", as it is used herein, relates to any pharmacologically active substance having the property of reducing blood triglyceride levels or triglyceride levels in other tissues.

The term "antiepileptic or anticonvulsant", as it is used herein, relates to the attenuation of epileptic or convulsive seizures, for example, in the duration and/or in the intensity, or to the disappearance of epileptic or convulsive seizures, or to the reduction or disappearance of the side effects thereof.

The term "biosafe" as it is used herein, relates to the absence of toxic effects, generation of tumors, alterations in embryologic development (teratogenesis) or other adverse effects.

The term "neurodegenerative disease", as it is used herein, includes diseases which result from the degeneration or deterioration of nervous tissue, particularly of neurons, leading over time to a dysfunction or to a disability; the term degeneration includes loss of cell viability, loss of cell function and/or loss of the number of cells (neurons or others). Illustrative, non-limiting, examples of neurodegenerative diseases include Alzheimer's disease, Huntington's disease, Parkinson's disease, amyotrophic lateral sclerosis (ALS), multiple sclerosis, etc. In a particular embodiment, said neurodegenerative disease is a disease related to neuronal death caused by a substance which, for example, causes oxidative stress or endoplasmic reticulum stress or apoptosis or excitotoxicity or neuronal death in general.

The term "disease associated with undesired oxidation", as it is used herein, relates to a disease caused by undesired oxidation (e.g., excessive oxidation) or in which said undesired oxidation is a symptom. Said undesired oxidation can be a result of the damage caused by free radicals on proteins, DNA and/or lipids independently from the specific free radical involved or from the target. Undesired oxidation involves an excessive generation of free radicals which can cause a dysfunction in cells, tissues or organs and can therefore form a potential mechanism of a disease. In a particular embodiment, said undesired oxidation can be caused by age (aging) or by a neurodegenerative process and can cause by itself or in combination with other factors the onset of several diseases. In a specific embodiment, said undesired oxidation relates to the oxidative damage caused by a substance which causes oxidative stress.

The term "age-associated pathological process", as it is used herein, relates to any age-related event or combination of events causing loss of cell viability of the nervous tissue or cell sensitization of the nervous tissue, loss of cell function and/or loss of the number of cells (neurons or others), including cell metabolic dysfunction, stress processes, infections by pathogens, genetic alterations, genetic susceptibility, trauma, ischemia, epilepsy, etc.

The term "cardiovascular disease", as it is used herein, relates to any disease or dysfunction or alteration of the heart or of the rest of the cardiovascular system or of the blood.

The term "epilepsy", as it is used herein, relates to a chronic brain syndrome having varied causes, characterized by recurrent seizures due to excessive hypersynchronic discharges of nervous impulses by the brain neurons, associated eventually with several clinical and paraclinical manifestations. The seizures can be convulsive or non-convulsive. Epilepsy can have many causes; in some cases it can be due to different types of brain injuries (e.g., brain traumas, sequelae of meningitis, tumors, etc.); in other cases there is no injury but a genetic predisposition to seizures; in other cases, the etiology of the epilepsy can be environmental, due to pharmacological treatments, due to excitotoxicity, trauma, stress processes, aging, development problems, neurological diseases, psychological crises, problems during gestation, problems during labor, etc.

The term "epileptic or convulsant", as it is used herein, relates to any epileptic seizure or convulsion of any etiology, for example, genetic, environmental, due to pharmacological treatments, due to excitotoxicity, due to trauma, due to stress processes, due to aging, due to development problems, due to neurological diseases, due to psychological crises, due to problems during gestation, due to problems during labor, etc. An epileptic seizure occurs when an abnormal electrical activity in the brain causes an involuntary change of body movement or function, feeling, in the capacity of being alert or in behavior, and can be partial or generalized (convulsive or non-convulsive).

The term "cognitive deterioration", as it is used herein, relates to the loss or alteration of mental functions, such as memory, orientation, language, visual recognition or conduct which interfere with the social activity and interaction of the person affected persistently over time.

The term "fungal or viral infections", as it is used herein, relates to any colonization of a microscopic fungus or virus which is harmful for the normal functioning or for the survival of the colonized organism or host.

The term "subject", as it is used herein, relates to a member of a mammal species and includes but is not limited to domestic animals, primates and humans; preferably, the subject is a male or female human being of any age or race. In a particular embodiment, said subject is a mammal which suffers, or is susceptible of suffering, age-associated pathological processes, such as aging, or a neurodegenerative disease, such as a chronic neurodegenerative disease.

The term "pharmaceutically acceptable", as it is used herein, relates to the fact that the compound is physiologically tolerable and generally does not cause an allergic reaction or a similar unfavorable reaction, such as a gastric disorder, dizziness or the like, when administered to a subject; said term "pharmaceutically acceptable" preferably means approved by a government regulatory agency or listed in the United States Pharmacopoeia or in another generally recognized pharmacopoeia for use in animals (e.g., European Pharmacopoeia, etc.).

The term "pharmaceutically acceptable salt", as it is used herein, includes "pharmaceutically acceptable metal salts" as well as "pharmaceutically acceptable amine salts". The term "pharmaceutically acceptable metal salt" contemplates salts formed with sodium, potassium, calcium, magnesium, aluminum, iron or zinc ions. The term "pharmaceutically acceptable amine salt" contemplates salts with ammonia and organic nitrogen bases strong enough to form salts with carboxylic acids. Said pharmaceutically acceptable salts can be obtained by conventional methods known by persons skilled in the art.

The compound (1S,2S,6R,8S,8aR)-1,2,6,7,8,8a-hexahydro-3,7-dimethyl-8-[2-[(2R,4R)-tetrahydro-4-hydroxy-6-oxo-2H-pyran-2-yl]ethyl]-1-naphthalenyl-2-ethyl-butyrate can be obtained by semisynthesis methods, such as those described for other compounds of the same family in United States patent US 4866090.

A number of assays performed by the inventors have clearly shown the neuroprotective effect of compound NST0037 against the action of a substance causing oxidative stress, as well as its neuroprotective effect against the action of a substance causing endoplasmic reticulum stress, and its neuroprotective effect against the action of a substance causing apoptosis in human cholinergic neurons, as well as its neuroprotective effect against a substance causing excitotoxicity, oxidative damage, apoptosis, hippocampal atrophy, neuronal death, cognitive deterioration, temporal memory loss, spatial memory loss, etc.

The neuroprotective effect against the action of a substance causing oxidative stress, such as xanthine/xanthine oxidase (XXO), is described in Example 2. It is observed in said example that compound NST0037 is capable of significantly and quantitatively reducing neuronal death caused by oxidative stress, which clearly shows the neuroprotective capacity of this compound (Figure 1). For the purpose of better defining the neuroprotective effect of the compound, the inventors analyzed the neurodegenerative process with greater detail by means of the analysis of neuronal death caused by the action of a substance causing endoplasmic reticulum stress (tunicamycin), determining that compound NST0037 is capable of significantly and quantitatively reducing neuronal death caused by endoplasmic reticulum stress, which clearly shows the neuroprotective capacity of said compound (Figure 2). For the purpose of better defining the neuroprotective effect of the compound, the inventors analyzed the neurodegenerative process with greater detail by means of the analysis of neuronal death caused by the action of a substance causing apoptosis (camptothecin), determining that compound NST0037 is capable of significantly and quantitatively reducing neuronal death caused by apoptosis, which clearly shows the neuroprotective capacity of said compound (Figure 3). Likewise, for the purpose of better defining the neuroprotective effect of the compound the inventors analyzed the neurodegenerative process with greater detail by means of flow cytometry analysis of neuronal death caused by apoptosis and its inhibition by said compound in comparison with a specific inhibitor of neuronal death by apoptosis, Z-VAD-fmk, determining that compound NST0037 is capable of significantly and quantitatively inhibiting neuronal death caused by apoptosis (Figure 4).

For the purpose of better defining the neuroprotective effect of the compound, the inventors analyzed the neurodegenerative process with greater detail by means of the analysis of neuronal death caused by an excitotoxic substance (kainate) in the hippocampal neurons of mice, as described in Example 3. It is observed in said example that compound NST0037 is capable of significantly and quantitatively reducing neuronal death caused by an excitotoxic substance, which clearly shows the neuroprotective capacity of said compound (Figure 5). It is demonstrated in said chart that the treatment with NST0037 administered before and after, or only after, the administration of kainate causes protection of the hippocampal neurons, inhibiting neuronal death in the CA1 and CA2 areas. In contrast, the administration of compound NST0037 alone does not cause any considerable histopathological change, showing no noteworthy differences with the control group. Likewise, as is known, the death of hippocampal neurons induces the cognitive deterioration of the animal in some cases, affecting memory processes; for this reason, the inventors analyzed if the neuroprotective effect of the compound was accompanied by a reduction of the temporal and spatial memory loss caused by an excitotoxic substance. It is demonstrated in the obtained results on the temporal memory (Figure 6) that the treatment with NST0037 administered before and after, or only after, the administration of kainate causes protection against the loss of this type of memory. It is demonstrated in the obtained results on the spatial memory (Figure 7) that the treatment with NST0037 administered before and after, or only after, the administration of kainate causes protection against the loss of this type of memory. In contrast, the administration of compound NST0037 alone does not cause any noteworthy change in the cognitive state of the animals, showing no considerable differences with the control group. Likewise, as is known, the administration of an excitotoxic substance and neuronal death induces the death of the animal in some cases; for this reason, the inventors analyzed if the neuroprotective effect of compound NST0037 was accompanied by a reduction of the mortality caused by an excitotoxic substance. It is demonstrated in the obtained results (Figure 8) that the treatment with NST0037 administered before and after, or only after, the administration of kainate causes a higher survival rate, indicating that the treatment with said compound protects the animals from death and against the rest of the organic effects caused by an excitotoxic substance. In contrast, the administration of compound NST0037 alone does not cause any noteworthy variation on the survival rate of the animals, showing no considerable differences with the control group.

Likewise, as is known, the administration of an excitotoxic substance induces convulsive seizures and epilepsy in the animal in some cases; for this reason, the inventors analyzed if the neuroprotective effect of compound NST0037 was accompanied by an antiepileptic and anticonvulsant effect caused by an excitotoxic substance (Example 4), observing that the administration of NST0037 delayed the time of onset of the first convulsion (latency) (Figure 9), furthermore causing a reduction in the severity and frequency of the epileptic symptoms (Figure 10), which demonstrates the antiepileptic or anticonvulsant effect of compound NST0037.

Furthermore, and due to the nature of compound NST0037, the inhibitory capacity of the 3-hydroxy-3-methylglutaryl coenzyme A reductase (HMGR) enzyme was studied. Surprisingly, and as is shown in Example 5, the results demonstrate that compound NST0037 has an evident hypocholesterolemic effect. It can be seen in the obtained results and as shown in Figure 11 that the inhibitory activity of compound NST0037 on the HMGR enzyme is within the range of two of the commercial statins (atorvastatin and simvastatin) commonly used to reduce cholesterol levels in the human population. It is demonstrated here that compound NST0037 exerts an inhibitory effect on said enzyme very similar to the statin with proven higher activity (atorvastatin), and inhibits the HMGR enzyme 7.5-fold more than the safest statin (simvastatin).

For the purpose of better defining the hypocholesterolemic effect of the compound, the inventors analyzed the hypocholesterolemic activity with greater detail by means of the analysis of the variations of the plasma cholesterol fractions in mice with endogenous hypercholesterolemia, as described in Example 5. To that end, the effect of compound NST0037 and of simvastatin was compared in two groups of mice, the total cholesterol levels being determined after the administration of the compounds (Figure 12), in which it was surprisingly observed that both compounds caused a similar hypocholesterolemic effect. For the purpose of better defining the hypocholesterolemic effect of the compound, the cholesterol levels in the LDL, HDL and VLDL fractions were analyzed, being determined that both compounds similarly reduce cholesterol levels in the LDL and VLDL fractions, but not in the HDL fraction (Figures 13 to 15), which demonstrates a hypocholesterolemic and cardioprotective effect. For the purpose of better defining the hypocholesterolemic effect of the compound, the free and esterified cholesterol levels were analyzed, being determined that both compounds similarly reduce the esterified cholesterol levels, but not the free cholesterol levels (Figure 16 to 17), which shows a hypocholesterolemic and cardioprotective effect.

For the purpose of better defining the hypocholesterolemic effect of the compound, the inventors analyzed the hypocholesterolemic activity with greater detail by means of the analysis of the variations of plasma cholesterol fractions in mice with induced hypercholesterolemia, as is described in Example 6. To that end, the effect of compound NST0037 and of simvastatin was compared in two groups of mice, the total cholesterol levels being determined after the administration of the compounds (Figure 18), in which it was surprisingly observed that compound NST0037 had a higher hypocholesterolemic effect than simvastatin. For the purpose of better defining the hypocholesterolemic effect of the compound, the cholesterol levels in the LDL, HDL and VLDL fractions were analyzed, being determined that compound NST0037 more effectively reduces cholesterol levels in the LDL fraction than simvastatin, neither altering cholesterol levels in the HDL fraction (like simvastatin) or in the VLDL fraction (unlike simvastatin) (Figures 19 to 21), which demonstrates a hypocholesterolemic and cardioprotective effect. For the purpose of better defining the hypocholesterolemic effect of the compound, the free and esterified cholesterol levels were analyzed, being determined that in both cases NST0037 reduces both cholesterol fractions more effectively than simvastatin (Figures 22 and 23), which demonstrates a hypocholesterolemic and cardioprotective effect.

In order for a compound to be administered to the human population, it is necessary that its innocuousness and safety be demonstrated. For this purpose, the inventors analyzed the biosafety of compound NST0037 in a widely used toxicological model, the zebrafish embryo, following the methodology described in the OECD C15 protocol, as described in Example 7. In this model, the inventors compared the effect of the compound with simvastatin at concentrations greater than the doses used in clinical practice for the purpose of causing evident damage in the embryos in order to be able to better define the adverse effects of said compound. Thus, the administration of a high dose of NST0037 caused the mortality of all the embryos of the assay, as occurred with the same dose of simvastatin, although the latter was more toxic, since the reduction of the survival rate started earlier in time (Figure 24). When a dose that was 10 times lower was used, simvastatin caused a significant reduction of the survival rate in comparison with the controls, while NST0037 did not cause a statistically significant reduction of the survival rate of the embryos, indicating its higher safety (Figure 25). For the purpose of better defining the biosafety of the compound, the lethal doses 50 (LD₅₀) were studied at different time points with treatments of NST0037 or of simvastatin in a semistatic method, observing that at all times the LD₅₀ of simvastatin were lower than those of NST0037, indicating higher biosafety of the latter compound (Figure 26). For the purpose of better defining the biosafety of the compound, the percentage of healthy larvae at the end of the experiment was studied in comparison with simvastatin, a higher number of healthy larvae being observed in the treatments with NST0037 in comparison with simvastatin (Figure 27). For the purpose of better defining the biosafety of the compound, the percentage of larvae with malformations or with an anomalous appearance over time was studied in comparison with simvastatin, a lower number of larvae with malformations or with an anomalous appearance being observed in the treatments with NST0037 in comparison with simvastatin (Figure 28). For the purpose of better defining the biosafety of the compound, the percentage of heartbeats according to the doses used was studied, comparing NST0037 with simvastatin, a higher reduction of the cardiac rhythm being observed in the highest evaluated dose of simvastatin than in that of NST0037, whereas at lower doses only simvastatin caused a statistically significant reduction of the cardiac rhythm (Figure 29), indicating a higher biosafety of compound NST0037.

The antifungal activity of compound NST0037 was also studied by means of bioassay against statins (lovastatin, atorvastatin and simvastatin). The obtained results showed that compound NST0037 was the only one capable of causing inhibition halos in the entire assayed concentration range, even at the lowest concentrations (Figure 30), indicating a higher antifungal activity.

In addition, the capacity of compound NST0037 to increase seladin-1/DHCR24 gene expression was studied since the neuroprotective effects of the increased expression of this gene against Alzheimer's disease have been demonstrated (Cechi et al., J. Cell. Mol. Med. 2008; 12: 1990-2002). It has been described (Greeve et al., J. Neurosci. 2000; 20: 7345-52) that the product of the seladin-1/DHCR24 gene exerts its neuroprotective power by means of the antiapoptotic effect by inhibition of caspase-3, and regulating cholesterol synthesis from desmosterol, which determinates the generation of a barrier against the neurotoxic injuries and prevents the production of β-amyloid. These mechanisms of action indicate that the increase of seladin-1/DHCR24 gene expression has a general neuroprotective effect, therefore those drugs that cause an increase of the expression of this gene can potentially be used in the prevention and/or treatment of neuronal death associated to neurodegenerative diseases (e.g., Alzheimer's, Parkinson's, multiple sclerosis, amyotrophic lateral sclerosis, *status* epilepticus, Huntington's, etc.) or of diseases associated with undesired oxidation or of age-associated pathological processes. The capacity of increasing seladin-1/DHCR24 gene expression by means of the administration of NST0037 is described in Example 9. It is observed in said example that compound NST0037 is capable of increasing quantitatively, significantly and in a dose-dependent manner the seladin-1/DHCR24 gene expression, which is demonstrated both by means of gene expression analysis using microarray technology and by means of relative expression analysis using real time quantitative PCR. Said results clearly show the neuroprotective capacity of compound NST0037 (Figure 31). Additionally, the increase of seladin-1/DHCR24 gene expression and the increase in the amount of the protein coded by this gene have also been corroborated in a parallel study using simvastatin. The results indicate that this statin is also capable of increasing the expression of this gene, thus explaining its neuroprotective capacity, and indicating that it refers to a general mechanism of the statin family which is capable of increasing seladin-1/DHCR24 gene expression.

The pharmaceutical composition provided by this invention can contain compound NST0037, and/or its hydroxy acid form and/or a pharmaceutically acceptable salt of said hydroxy acid and/or a pharmaceutically acceptable prodrug or solvate of the compound or of its hydroxy acid form together with one or more pharmaceutically acceptable adjuvants, vehicles or excipients.

The term pharmaceutically acceptable "salt, prodrug or solvate" relates to any pharmaceutically acceptable salt, solvate or any other compound which is capable of providing (directly or indirectly) a compound as has been described in the present invention in its administration to the recipient. Nevertheless, pharmaceutically unacceptable salts also fall within the scope of the invention, since the latter can be useful for the preparation of pharmaceutically acceptable salts. The salts and prodrugs can be prepared by means of methods known in the state of the art.

Any compound which is a prodrug of the compound of formula (I) or of its hydroxy acid form is within the scope of the invention. The term "prodrug" is used in its broadest meaning and encompasses those derivates which are converted *in vivo* into the compounds of the invention. Such derivates would be evident to a person with average skilled in the art and include the following derivatives of the present compounds: esters, amino acid esters, phosphate esters, metal sulfonate salt esters, carbamates and amides. The compounds according to the invention can be in a crystalline form or as free compounds or as solvates (for example, hydrates) and it is intended that both forms are within the scope of the present invention. Solvation methods are generally known in the state of the art. In a particular embodiment the solvate is a hydrate.

The pharmaceutical compositions containing compound NST0037, or a hydroxy acid form thereof or a pharmaceutically acceptable salt of said hydroxy acid, can be formulated in any pharmaceutical dosage form suitable for its administration by the chosen administration route, e.g., oral, parenteral (subcutaneous, intramuscular, intravenous, intraperitoneal route, etc.), topical, rectal route, etc. By way of a non-limiting illustration, the pharmaceutical compositions provided by this invention can be formulated in a solid pharmaceutical dosage form administered by the oral route (e.g., granules, tablets, capsules, etc.), in a liquid pharmaceutical dosage form administered by the oral route (e.g., solutions, suspensions, emulsions, etc.), in a pharmaceutical dosage form administered by the parenteral route (e.g., solutions, suspensions, emulsions, etc.). To that end, in each case, the suitable pharmaceutically acceptable vehicles and excipients will be chosen for the chosen pharmaceutical dosage form and route of administration, for example, binding agents, diluents, disintegrating agents, lubricants, wetting agents, etc., for the formulation of solid pharmaceutical dosage forms, and buffers, surfactants, etc., for the formulation of liquid pharmaceutical dosage forms. Said vehicles and excipients must be pharmaceutically acceptable and pharmacologically tolerable and have to be able to be combined with other components of the formulation without exerting any adverse effect on the subject treated. Information on said vehicles and excipients, as well as on said pharmaceutical dosage forms of said active ingredient can be found in Galenic Pharmacy treatises. A review of the different pharmaceutical dosage forms of drugs, in general, and of their methods of preparation can be found in the book *"*Tratado de Farmacia Galenica" ("Galenic Pharmacy Treatise"), by C. Faulí i Trillo, 1st Edition, 1993, Luzán 5, S.A. de Ediciones.

The pharmaceutical composition provided by this invention comprises, compound NST0037, or a hydroxy acid form thereof or a pharmaceutically acceptable salt of said hydroxy acid, in a therapeutically effective amount. In the way used in this description, the expression "therapeutically effective amount" relates to the amount of compound calculated to cause the desired effect. The dose of compound NST0037, or a hydroxy acid form thereof or a pharmaceutically acceptable salt of said hydroxy acid, to be administered to a subject can vary within a wide range depending on a number of factors, including the characteristics of the compound used, e.g., its biological half-life and activity, the concentration of the compound in the pharmaceutical composition, the clinical situation of the subject, the severity of the pathology, the chosen pharmaceutical dosage form, etc.. The pharmaceutical composition provided by this invention can be administered one or more times a day for preventive or therapeutic purposes or, alternatively, others administration regimens can be followed, not necessarily daily but also at precise times, weekly, etc.

If desired, the pharmaceutical composition provided by this invention can be used together with other drugs, for example, drugs useful in the treatment of neurodegenerative diseases, cognitive deterioration, diseases associated with undesired oxidation, age-associated pathological processes and progeria, epilepsy, epileptic seizures, convulsions, cardiovascular diseases, or fungal or viral infections for the purpose of increasing the efficacy of the pharmaceutical composition provided by this invention, a combination therapy thus being generated. Said additional drugs can form part of the same pharmaceutical composition or, alternatively, can be provided as a separate pharmaceutical composition for its administration at the same time (simultaneous administration) as the pharmaceutical composition provided by this invention or at different times (sequential administration) with respect to the administration of the pharmaceutical composition provided by this invention.

The following examples serve to illustrate the invention and must not be considered as limiting thereof.

### EXAMPLE 1

### Synthesis of (1S,2S,6R,8S,8aR)-1,2,6,7,8,8a-hexahydro-3,7-dimethyl-8-[2-[(2R,4R)-tetrahydro-4-hydroxy-6-oxo-2H-pyran-2-yl]ethyl]-1-naphthalenyl-2-ethyl-butyrate

The compound identified as NST0037 was prepared following the methodology described in Hoffman, et al. (J. Med. Chem., 1986, 29, 849-852) for similar compounds.

### 1.1. Purification of lovastatin

Lovastatin was purified from an extract of natural origin by column chromatography using a hexane and ethyl acetate gradient as eluent.

### 1.2. Obtaining monacolin J

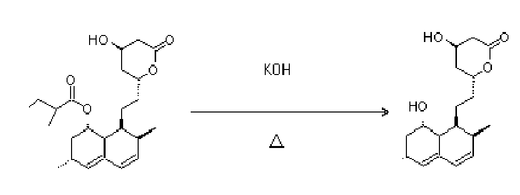

A solution of 0.7 g of potassium hydroxide in 0.5 ml of water is prepared and 3 ml of methanol are added little by little. 0.5 g of Lovastatin are subsequently added and the solution is placed under reflux for 21 hours. After the treatment of the reaction, a 50% mixture of monacolin J and the opened product is obtained.

### 1.3. Preparation of protected derivative

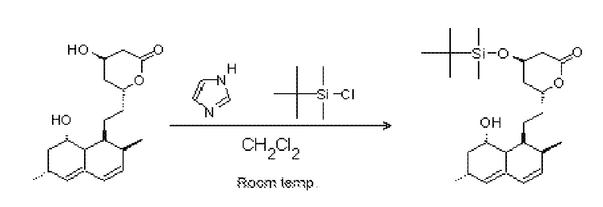

A solution of 0.5 g of Monacolin J in 10 ml of dichloromethane is prepared. 0.4345 g of imidazol are added and it is stirred until dissolution. Then 0.4835 g of tert-butyl-dimethylsilane chloride dissolved in 5 ml of dichloromethane are added, and stirring is continued for 24 hours. The reaction is followed by TLC using dichloromethane-methanol (10:1) as eluent. Yield: 96 %.

### 1.4. Preparation of the acylated derivative

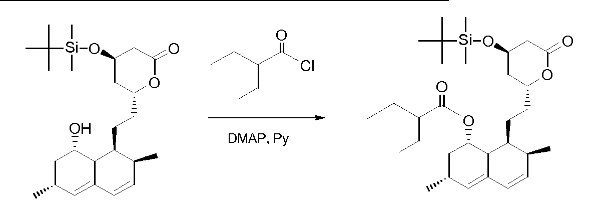

0.3 g of the protected derivative previously obtained are dissolved in a flask with inert atmosphere in 2 ml of pyridine. 0.06 g of DMAP dissolved in 2 mL of pyridine are subsequently added. The reaction flask is placed in an ice bath and 0.378 ml of 2-ethylbutyryl chloride are added. Then it is stirred for one hour at 0°C and at room temperature for 18 hours. The reaction is followed by TLC using hexane-ethyl acetate (2:1) as eluent. Yield: 95%.

### 1.5. Synthesis of the final compound

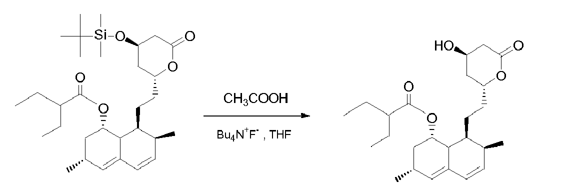

0.368 g of the derivative previously obtained are dissolved in 2 ml of THF. Then a solution of 0.16 ml of acetic acid and 2.16 ml of 1M tetrabutylammonium fluoride is added to the reaction medium. The reaction mixture is stirred at room temperature for 16 hours. The reaction is followed by TLC using dichloromethane-acetone (6:1) as eluent. Yield: 75 %.

### EXAMPLE 2

### Protection by NST0037 against neuronal death induced by different aggressions: oxidative stress, endoplasmic reticulum stress and apoptosis

### 2.1. Protection by NST0037 against neuronal death induced by oxidative stress

The assay was performed on human neuroblastoma SK-N-MC cells in culture from the American Type Culture Collection (ATCC), in all cases strict rules of sterility were followed and the manipulation was performed in class II biological safety cabinets following European standard EN 12469. The cells were maintained in the following culture medium: Minimum Essential Medium Eagle (MEM) supplemented with 1 mM sodium pyruvate, 2 mM L-glutamine, 0.1 mM non-essential amino acids, 0.05 mg/ml gentamicin and 10% fetal bovine serum.

The inhibition caused by compound NST0037 of cell death caused by treatment with xanthine/xanthine oxidase which generates oxidative damage (causes free radicals such as hydrogen peroxide, superoxide anion, hydroxyl radical), which triggers cell death, was analyzed. These cells, not exceeding 15 passages, were seeded on 96-well plates treated for adherent cells with a cell concentration of 5x10⁴ cells/well; 3 wells of the plate were seeded for each condition of the assay.

After 24 hours of cell incubation at 37°C and 5% CO₂, the cell treatments were performed with 100 µl of total volume for the following conditions:
- Control: culture medium (medium)
- Xanthine/xanthine oxidase (XXO): medium plus 10 µM xanthine / 60 mU/mL xanthine oxidase, causing the death of 50% of the cells.
- XXO plus NST0037: medium plus XXO (10 µM / 60 mU/mL) plus NST0037 at 1, 4, 10, 20, 40 or 100 µM.

The cells were incubated (at 37°C and 5% CO₂) with these treatments for 22 hours, after which time the WST-1 reagent (Roche) was added. The Test WST-1 is based on the measurement of metabolic activity. Cell damage causes the loss of the ability of cells to obtain the energy necessary to maintain their metabolic functions and cell growth, therefore the metabolically active (live) cells reduce tetrazolium salt to formazan by means of the succinate-tetrazolium reductase system (of the mitochondrial respiratory chain). The formazan which is formed can be detected colorimetrically since it has an absorbance of 440 nm. The reading was taken in a plate reader at 440 nm 2 hours after adding the reagent.

The obtained results are shown, as can be seen in Figure 1, as the percentage of cell death for each treatment relating to death caused by XXO. Protection against death was observed at from 1 to 100 µM of NST0037, the differences with respect to XXO being statistically significant, according to the Student's t-test, for all the concentrations evaluated, reaching maximum protection of 78% at 20 µM. These results indicate that compound NST0037 shows a protective effect against the death of human cells of neuronal origin caused by oxidative stress.

### 2.2. Protection by NST0037 against neuronal death induced by endoplasmic reticulum stress

The assay was performed on human neuroblastoma SK-N-MC cells in culture from the American Type Culture Collection (ATCC), in all cases strict rules of sterility were followed and the manipulation was performed in class II biological safety cabinets following European standard EN 12469. The cells were maintained in the following culture medium: Minimum Essential Medium Eagle (MEM) supplemented with 1 mM sodium pyruvate, 2 mM L-glutamine, 0.1 mM non-essential amino acids, 0.05 mg/ml gentamicin and 10% fetal bovine serum.

The inhibition caused by compound NST0037 of cell death caused by treatment with tunicamycin generating reticular stress was analyzed. Tunicamycin is an inhibitor of protein N-glycosylation, causing abnormal protein folding in the endoplasmic reticulum, therefore said proteins are accumulated and cause stress, resulting in cell death. These cells, not exceeding 15 passages, were seeded on 96-well plates treated for adherent cells with a cell concentration of 5x10⁴ cells/well; 3 wells of the plate were seeded for each condition of the assay.

After 24 hours of cell incubation at 37°C and 5% CO₂, the cell treatments were performed with 100 µl of total volume for the following conditions:
- Control: culture medium (medium)
- Tunicamycin (Tm): medium plus tunicamycin 24 µM, causing the death of 50% of the cells.
- Tm plus NST0037: medium plus Tm (24 µM) plus NST0037 at 1, 4, 10, 40 or 100 µM.

The cells were incubated (at 37°C and 5% CO₂) with these treatments for 22 hours, after which time WST-1 reagent (Roche) was added. The WST-1 Test is based on the measurement of metabolic activity. Cell damage causes the loss of the ability of cells to obtain the energy necessary to maintain their metabolic functions and cell growth; therefore the metabolically active (live) cells reduce tetrazolium salt to formazan by means of the succinate-tetrazolium reductase system (of the mitochondrial respiratory chain). The formazan that is formed can be detected colorimetrically since it has an absorbance of 440 nm. The reading was taken in a plate reader at 440 nm 2 hours after adding the reagent.

The obtained results are shown, as can be seen in Figure 2, as the percentage of cell death for each treatment relating to death caused by Tm. Protection against death was observed at from 1 to 100 µM of NST0037, reaching 55% at 40 µM. These results indicate that compound NST0037 shows a protective effect against the death of human cells of neuronal origin caused by endoplasmic reticulum stress.

### 2.3. Protection by NST0037 against neuronal death induced by apoptosis

The assay was performed on human neuroblastoma SK-N-MC cells in culture from the American Type Culture Collection (ATCC), in all cases strict rules of sterility were followed and the manipulation was performed in class II biological safety cabinets following European standard EN 12469. The cells were maintained in the following culture medium: Minimum Essential Medium Eagle (MEM) supplemented with 1 mM sodium pyruvate, 2 mM L-glutamine, 0.1 mM non-essential amino acids, 0.05 mg/ml gentamicin and 10% fetal bovine serum.

The inhibition caused by compound NST0037 of cell death caused by treatment with camptothecin generating cell cycle arrest was analyzed. Camptothecin is an inhibitor of Topoisomerase I, it therefore prevents DNA duplication and triggers cell cycle arrest and death due to apoptosis. These cells, not exceeding 15 passages, were seeded on 96-well plates treated for adherent cells with a cell concentration of 5x10⁴ cells/well; 3 wells of the plate were seeded for each condition of the assay.

After 24 hours of cell incubation at 37°C and 5% CO₂, the cell treatments were performed with 100 µl of total volume for the following conditions:
- Control: culture medium (medium)
- Camptothecin (Campto): medium plus camptothecin 20 nM, causing death of 50% of the cells.
- Camptothecin plus NST0037: medium plus camptothecin (20 nM) plus NST0037 at 1, 4, 10, 20, 40 or 100 µM.

The cells were incubated (at 37°C and 5% CO₂) with these treatments for 22 hours, after which time the WST-1 reagent (Roche) was added. The Test WST-1 is based on the measurement of metabolic activity. Cell damage causes the loss of the ability of cells to obtain the energy necessary to maintain their metabolic functions and cell growth, therefore the metabolically active (live) cells reduce tetrazolium salt to formazan by means of the succinate-tetrazolium reductase system (of the mitochondrial respiratory chain). The formazan which is formed can be detected colorimetrically since it has an absorbance of 440 nm. The reading was taken in a plate reader at 440 nm 2 hours after adding the reagent.

The obtained results are shown, as can be seen in Figure 3, as the percentage of cell death for each treatment relating to death caused by camptothecin. Protection against death was observed at from 4 to 100 µM of NST0037, reaching 18%, 28%, 27% and 27% at 4, 10, 40 and 100 µM, respectively. These results indicate that compound NST0037 shows a protective effect of the death of human cells of neuronal origin caused by cell cycle arrest.

### 2.4. Determination of the inhibition of apoptosis by means of flow cytometry

The assay was performed on human neuroblastoma SK-N-MC cells in culture from the American Type Culture Collection (ATCC), in all cases strict rules of sterility were followed and the manipulation was performed in class II biological safety cabinets following European standard EN 12469. The cells were maintained in the following culture medium: Minimum Essential Medium Eagle (MEM) supplemented with 1 mM sodium pyruvate, 2 mM L-glutamine, 0.1 mM non-essential amino acids, 0.05 mg/ml gentamicin and 10% fetal bovine serum.

The inhibition caused by compound NST0037 against apoptosis (programmed cell death) caused by treatment with camptothecin which inhibits the enzyme Topoisomerase I, which prevents DNA duplication and triggers apoptotic cell death, was analyzed. These cells, not exceeding 15 passages, were seeded on 6-well plates treated for adherent cells with a cell concentration of 8x10⁵ cells/well; 2 wells of the plate were seeded for each condition of the assay.

After 24 hours of cell incubation at 37°C and 5% CO₂, the cell treatments were performed with 2 ml of total volume for the following conditions:
- Control: culture medium (medium)
- Camptothecin: medium plus camptothecin 50 µM.
- Camptothecin plus Z-VAD-fmk: medium plus 50 µM camptothecin plus 50 µM Z-VAD-fmk, as positive inhibition control.
- Camptothecin plus NST0037: medium plus 50 µM camptothecin plus NST0037 at 10, 40 or 100 µM.
   The cells were incubated (at 37°C and 5% CO₂) with these treatments for 6 hours, after which time they were collected along with their culture medium and centrifuged at 300xg for 5 minutes. The medium was removed, a washing was performed with PBS and the cells were fixed for 2 minutes with 500 µl of 70% ethanol at -20°C. Once fixed, they were centrifuged at 400xg for 5 minutes, washed with PBS and 0.05 mg/ml propidium iodide, diluted in cycling buffer (0.1% sodium citrate, 0.3% Nonidet P-40 and 0.02 mg/ml RNase) were added and they were incubated for 1 hour at 37°C. After this time they were analyzed by flow cytometry, comparing the fluorescence of propidium iodide against the amount of DNA. The percentage of apoptosis was measured on the sub-G1 region of each of the conditions.

The obtained results are shown, as can be seen in Figure 4, as the percentage of the inhibition of apoptosis of each treatment relating to apoptosis caused by camptothecin. Maximum protection of 18% was observed at 40 and 100 µM of NST0037, therefore this compound shows a protective effect of apoptosis in human cells of neuronal origin. Z-VAD-fmk, a specific inhibitor of caspases, inhibits the apoptosis caused by 32%.

### EXAMPLE 3

### Protection by NST0037 against neuronal death in the hippocampus, against cognitive deficit and against death caused by an excitotoxic substance

### 3.1. Protective effect of NST0037 against neuronal death in the hippocampus of mice caused by an excitotoxic substance

Based on the results of Example 2, the inventors decided to investigate if the neuroprotective effect of NST0037 demonstrated in human cholinergic neurons was corroborated in a model of sporadic Alzheimer's disease in mice by means of the administration of kainate (KA).

All the animals included for the experimental process were 12-week old males of the FVB/NHan strain. The experiments were conducted strictly following the *Guidance on the Operation of Animals (Scientific Procedures, Act. 1986*). The animals had their respective quarantine period and were treated with maximum precaution to minimize possible contaminations for inoculations and handling.

Twenty-eight animals were used in this assay and the treatments were all conducted by intraperitoneal (i.p.) route with a volume of 100 µl, according to the following regimens:
i) PBS+PBS+PBS regimen: 4 animals were initially treated with PBS with a daily dose for 2 days, on the second day the animals were inoculated with a new dose of PBS and for the next 7 days with a daily dose of PBS.
ii) PBS+KA+PBS regimen: 6 animals were initially treated with PBS with a daily dose for 2 days, on the second day the animals were inoculated with a dose of kainate of 25 mg/Kg and for the next 7 days with a daily dose of PBS.
iii) PBS+KA+NST0037 regimen: 7 animals were initially treated with PBS with a daily dose for 2 days, on the second day the animals were inoculated with a dose of kainate at 25 mg/Kg and for the next 7 days with a daily dose of NST0037 at 50 mg/kg.
iv) NST0037+KA+NST0037 regimen: 6 animals were initially treated with a daily dose for 2 days with NST0037 at 50 mg/kg, on the second day the animals were inoculated with a dose of kainate at 25 mg/Kg and for the next 7 days with a daily dose of NST0037 at 50 mg/kg.
v) NST0037+PBS+NST0037 regimen: 5 animals were initially treated with a daily dose for 2 days with NST0037 at 50 mg/kg, on the second day the animals were inoculated with a dose of PBS and for the next 7 days with a daily dose of NST0037 at 50 mg/kg.

Once the treatment time of 7 days ended, the animals were sacrificed and the brains were dissected. The brain samples were processed and included in paraffin. Hematoxylin and eosin stain was used in 5 µm thick sections to analyze the cell architecture of the hippocampus.

As shown in Figure 5, the PBS+KA+PBS administration regimen caused severe neuronal damage in the CA1 and CA2 regions of the hippocampus in mice. In the samples of said group, an absence of neurons, evidence of necrosis and a number of pycnotic nuclei were observed, which demonstrates neuronal death. However, the samples of the NST0037+KA+NST0037 group showed a cell structure identical to the controls (PBS+PBS+PBS group) without evidence of cell damage in the CA1 and CA2 regions. Furthermore and surprisingly, the samples of the PBS+KA+NST0037 group showed several signs of damage and neuronal death in the hippocampus. However, comparing *de visu* the samples of the PBS+KA+PBS group with those of the PBS+KA+NST0037 group, a higher number of neurons of the hippocampus without damage was observed in the group with the NST0037 treatment, indicating its neuroprotective effect. Finally, the samples of the NST0037+PBS+NST0037 group showed a pattern identical to that observed in the animals of the PBS+PBS+PBS group.

In summary, the pre-treatment with NST0037 (NST0037+KA+NST0037 group) completely protected against neuronal death caused by KA in the hippocampal CA1 and CA2 regions. Furthermore, the beginning of treatment with NST0037 after the inoculation of KA qualitatively reduced neuronal death and damage in this region. It was also demonstrated that the daily administration of NST0037 for 8 days was not neurotoxic for the mice.

### 3.2. Protective effect of NST0037 against episode-type memory deterioration in mice caused by an excitotoxic substance

The excitotoxicity caused by KA induces, a few days after its inoculation in mice, episode-type memory deterioration, affecting the temporal memory and causing a severe spatial memory deficit. As a result the inventors decided to investigate if the neuroprotective effect of NST0037 was accompanied by a protection of the memory which deteriorates due to the effect of an excitotoxic substance.

All the animals included for the experimental process were 12-week old males of the FVB/NHan strain. The experiments were conducted strictly following the *Guidance on the Operation of Animals (Scientific Procedures, Act. 1986*). The animals had their respective quarantine period and were treated with maximum precaution to minimize possible contaminations for inoculations and handling.

Twenty-eight animals were used in this assay and the treatments were all conducted by intraperitoneal (i.p.) route with a volume of 100 µl, according to the following regimens:
i) PBS+PBS+PBS regimen: 4 animals were initially treated with PBS with a daily dose for 2 days, on the second day the animals were inoculated with a new dose of PBS and for the following 3 days with a daily dose of PBS.
ii) PBS+KA+PBS regimen: 6 animals were initially treated with PBS with a daily dose for 2 days, on the second day the animals were inoculated with a dose of kainate at 25 mg/Kg and for the following 3 days with a daily dose of PBS.
iii) PBS+KA+NST0037 regimen: 7 animals were initially treated with PBS with a daily dose for 2 days, on the second day the animals were inoculated with a dose of kainate at 25 mg/Kg and for the following 3 days with a daily dose of NST0037 at 50 mg/kg.
iv) NST0037+KA+NST0037 regimen: 6 animals were initially treated with a daily dose for 2 days with NST0037 at 50 mg/kg, on the second day the animals were inoculated with a dose of kainate at 25 mg/Kg and for the following 3 days with a daily dose of NST0037 at 50 mg/kg.
v) NST0037+PBS+NST0037 regimen: 5 animals were initially treated with a daily dose for 2 days with NST0037 at 50 mg/kg, on the second day the animals were inoculated with a dose of PBS and for the following 3 days with a daily dose of NST0037 at 50 mg/kg.

Three days after inoculation of KA, an object recognition test called integral memory test was performed on the animals. Figure 6 shows the results of the relation in the time of the exploration of old objects compared to new objects (temporal memory). It was observed that the group with the NST0037+KA+NST0037 treatment regimen prevented the reduction in the temporal memory capacity in comparison with the mice of the PBS+KA+PBS group. Furthermore, the animals of the PBS+KA+NST0037 group also showed an improvement of the state of the temporal memory with respect to those treated in the PBS+KA+PBS group. The data also showed that the group of mice with the NST0037+PBS+NST0037 regimen also showed an improvement of the temporal memory with respect to the PBS+PBS+PBS group.

Figure 7 shows the results of the relation over time of the exploration of the displaced old object compared with the non-displaced old object (spatial memory). The group of mice with the NST0037+KA+NST0037 or NST0037+PBS+NST0037 treatment regimens showed a relation of the exploration of objects demonstrating that the spatial memory is intact since no differences were observed with respect to the PBS+PBS+PBS group. However, the mice of the PBS+KA+PBS group showed severe deterioration of spatial memory. The PBS+KA+NST0037 treatment group showed an improvement of this type of memory with respect to those that were treated in the PBS+KA+PBS group.

These studies indicate that the treatment before and after (or only after) the damage with an excitotoxic substance with compound NST0037 has a protective effect on episodic (temporal and spatial) memory which degenerates after the administration of an excitotoxic substance.

### 3.3. Protective effect of NST0037 against death caused by an excitotoxic substance

It is known that the administration of an excitotoxic substance to animals induces, in some cases, death of the animal. As a result, the inventors decided to investigate if the neuroprotective effect of NST0037 was accompanied by a reduction of the mortality caused by an excitotoxic substance.

All the animals included for the experimental process were 12-week old males of the FVB/NHan strain. The experiments were conducted strictly following the *Guidance on the Operation of Animals (Scientific Procedures, Act. 1986*). The animals had their respective quarantine period and were treated with maximum precaution to minimize possible contaminations for inoculations and handling.

Twenty-eight animals were used in this assay and the treatments were all conducted by intraperitoneal (i.p.) route with a volume of 100 µl, according to the following regimen:
vi) PBS+PBS+PBS regimen: 4 animals were initially treated with PBS with a daily dose for 2 days, on the second day the animals were inoculated with a new dose of PBS and for the following 3 days with a daily dose of PBS.
vii) PBS+KA+PBS regimen: 6 animals were initially treated with PBS with a daily dose for 2 days, on the second day the animals were inoculated with a dose of kainate at 25 mg/Kg and for the following 3 days with a daily dose of PBS.
viii) PBS+KA+NST0037 regimen: 7 animals were initially treated with PBS with a daily dose for 2 days, on the second day the animals were inoculated with a dose of kainate at 25 mg/Kg and for the following 3 days with a daily dose of NST0037 at 50 mg/kg.
ix) NST0037+KA+NST0037 regimen: 6 animals were initially treated with a daily dose for 2 days with NST0037 at 50 mg/kg, on the second day the animals were inoculated with a dose of kainate at 25 mg/Kg and for the following 3 days with a daily dose of NST0037 at 50 mg/kg.
x) NST0037+PBS+NST0037 regimen: 5 animals were initially treated with a daily dose for 2 days with NST0037 at 50 mg/kg, on the second day the animals were inoculated with a dose of PBS and for the following 3 days with a daily dose of NST0037 at 50 mg/kg.

The deaths were recorded during the entire time the assay lasted, and the results are shown in Figure 8 by means of Kaplan-Meier survival curves. The results of the study of the mortality caused by KA showed that the group with the NST0037+KA+NST0037 treatment regimen was protected against death associated to the damage caused by an excitotoxic substance. Furthermore, and more importantly, the survival of the animals in group with the PBS+KA+NST0037 treatment regimen increases in comparison with that of the group with the PBS+KA+PBS regimen.

These studies indicate that the treatment before and after (or only after) of the damage with an excitotoxic substance with compound NST0037 has a protective effect against mortality caused by the administration of an excitotoxic substance.

### EXAMPLE 4

### Antiepileptic effect of NST0037 against the action of an excitotoxic substance

It is known that the administration of an excitotoxic substance to animals induces, in some cases, epileptic seizures and convulsions in the animals. As a result, the inventors decided to investigate if the neuroprotective effect of NST0037 was accompanied by an antiepileptic effect caused by an excitotoxic substance.

All the animals included for the experimental process were 12-week old males of the FVB/NHan strain. The experiments were conducted strictly following the *Guidance on the Operation of Animals (Scientific Procedures, Act. 1986).* The animals had their respective quarantine period and were treated with maximum precaution to minimize possible contaminations for inoculations and handling.

The animals were intraperitoneally inoculated with 25 mg/kg of kainate (KA) dissolved in PBS. Thirteen animals were pre-treated 24 and 0.5 hours before inoculation with KA by means of intraperitoneal injection with PBS (PBS+KA administration regimen) and 6 were pre-treated 24 and 0.5 hours before inoculation with KA by means of intraperitoneal injection with NST0037 at a dose of 50 mg/kg (NST0037+KA administration regimen). After the inoculation, the animals were individually housed in trays to monitor them. The maximum epilepsy level of the animals was recorded during the observation according to the Racine scale every ten minutes and for at least 120 minutes post-inoculation (m.p.i.).

Comparative studies of the epileptogenic phenomena between the treatment with PBS (PBS+KA) and with NST0037 (NST0037+KA) were subsequently conducted. Differences were observed in the percentage of animals that entered in *status epilepticus,* i.e., they showed tonic-clonic seizures, among the NST0037+KA group which was 33.3% (2 out of 6) compared to the PBS+KA treatment regimen group which was 76.9% (10 out of 13), which demonstrates that compound NST0037 is antiepileptic and anticonvulsant. Differences over time were also observed in which the first convulsions occurred (*latency period*), in which the latency of the NST0037+KA group (103.3 ± 13.1 minutes) was greater than with the PBS+KA treatment regimen (74.6 ± 8.6 minutes) as shown in Figure 9, which demonstrates an antiepileptic and anticonvulsant effect of compound NST0037. Based on these results the inventors decided to investigate if the antiepileptic and anticonvulsant effect was confirmed with the epilepsy level and by the severity of the symptoms, observing that the animals of the group with the PBS+KA regimen reached epilepsy level 4 on the Racine scale, whereas the NST0037+KA regimen did not cause at any time an epilepsy level 3 on said scale, indicating that compound NST0037 is antiepileptic and anticonvulsant. Furthermore, based on the 100 m.p.i. the NST0037+KA regimen caused the disappearance of the epileptogenic symptoms, whereas the PBS+KA regimen showed severe seizures and spasms.

These studies indicate that treatment with compound NST0037 has an antiepileptic and anticonvulsant effect due to the administration of an excitotoxic substance.

### EXAMPLE 5

### Inhibitory activity of HMGR and hypocholesterolemic effect of NST0037 in an endogenous hyperlipidemia model in mice

### 5.1. Inhibitory effect of NST0037 on the HMGR enzyme activity

Due to the nature of the compound, the degree of inhibition of the HMGR enzyme by compound NST0037 was determined since this enzyme is key in cellular cholesterol synthesis and in the physiological regulation of said synthesis. The effect of this compound was compared with that of two known statins, atorvastatin and simvastatin, compounds for which a potent inhibitory effect on HMGR has already been described. For the reaction, HMGR uses NADPH as a reducing agent and 3-hydroxy-3-methylglutaryl coenzyme A (HMGCoA) as a substrate, producing mevalonic acid, CoASH and NADP+. The reduction of the absorbance at which NADPH (340 nm) is absorbed is a direct measurement of the catalytic activity of HMGR and serves to calculate the inhibition percentages of different compounds. In order to take the HMGR inhibition, the assay was performed on a 96-well plate, with a total reaction volume of 200 µl. The reaction buffer (50 mM KH₂PO₄, 1 M KCl, 2 mg/ml Bovine Serum Albumin [BSA] and 5 mM DTT, at pH=7.3) was prepared at the time the reaction was conducted and was maintained at 37°C. The following was included in the assays:
- A blank: Lacking HMGR which reports on the stability of NADPH for the reaction.
- A control: Contains all the components of the reaction but lacks the test compound.
- Test compounds: NST0037, Atorvastatin and Simvastatin at several concentrations. The acid form of the compounds is used in all the cases.

Five independent assays were conducted with NST0037, four independent assays with atorvastatin and six independent assays with simvastatin, the inhibition potency being determined in a range of concentrations which allowed defining the 50% inhibition constants (IC₅₀) by means of spectrophotometric determination of the drop of NADPH at 37°C, and as is shown in Figure 11. The reduction of absorbance at 340 nm allows calculating the percentage of HMGR enzyme activity with respect to the control. The IC₅₀ were determined by means of the Trimmed Spearman-Karber method (Version 1.5). The results indicate that the inhibition potency of NST0037 is surprisingly close to that of more potent statin, atorvastatin, and is 7.5 times more potent than simvastatin, which demonstrates a clear inhibitory effect of HMGR.

### 5.2. Hypocholesterolemic effect of NST0037 in an endogenous hyperlipidemia model (familial)

Based on the results of the previous point, the inventors decided to investigate if the inhibitory activity of HMGR enzyme by compound NST0037 corresponds with a variation of total cholesterol and of its fractions in an endogenous hyperlipidemia model in mice.

All the animals included for the experimental process were 46-week old males of the ApoB100 strain, which strain has a deficiency in removing cholesterol from the blood which causes an abnormally high increase of plasma cholesterol. The experiments were conducted strictly following the *Guidance on the Operation of Animals (Scientific Procedures, Act. 1986).* The animals had their respective quarantine period and were treated with maximum precaution to minimize possible contaminations for inoculations and handling.

In order to conduct the experiment on the fasting animals, blood was extracted by ocular puncture. The plasma was obtained from said blood, in which plasma total cholesterol levels and levels of its fractions were determined prior to administration of the substances under study. Immediately after that, the animals were intraperitoneally inoculated with 50 mg/kg of each of the test compounds. Four animals were treated with the vehicle and were considered the control group. A second group of five mice received 50 mg/Kg of simvastatin. Finally, a third group of five mice received 50 mg/Kg of NST0037. Twelve hours after the treatments, blood was again extracted from the fasting animals and the plasma was obtained from said blood. Figure 12 shows the plasma cholesterol levels at the initial time (t0) and twelve hours after the administration of the vehicle, simvastatin or NST0037 in the different groups of mice (t12), demonstrating that both compounds have a significant hypocholesterolemic effect. In relation to the different cholesterol fractions, both compounds considerably reduced the low density lipoprotein cholesterol (LDL-c) and very low density lipoprotein cholesterol (VLDL-c) levels without changing the high density lipoprotein cholesterol (HDL-c) levels, as shown in Figures 13 to 15. In relation to the free cholesterol (FC) and esterified cholesterol (EC) levels, both compounds reduced the EC levels in a similar manner without changing FC levels, as shown in Figures 16 and 17.

The results shown in this section demonstrate that compound NST0037 shows an effect that is surprisingly similar to simvastatin, significantly reducing TC, LDL-c, VLDL-c and EC levels without changing HDL-c or FC levels.

### EXAMPLE 6

### Hypocholesterolemic effect of NST0037 in an induced hyperlipidemia model in mice

Based on the results obtained in the experiment with ApoB100 animals, the inventors decided to investigate if compound NST0037 also showed a hypocholesterolemic effect in an induced acute hyperlipidemia model.

All the animals included for the experimental process were wild-type 6-week old male mice of the C57BL6 strain. The experiments were conducted strictly following the *Guidance on the Operation of Animals (Scientific Procedures, Act. 1986).* The animals had their respective quarantine period and were treated with maximum precaution to minimize possible contaminations for inoculations and handling.

In order to conduct the experiment on the fasting animals, blood was extracted by ocular puncture. The plasma was obtained from said blood, in which plasma total cholesterol levels and levels of its fractions were determined prior to administration of the substances under study. Immediately after that, the animals were intraperitoneally inoculated with 500 mg/kg of Triton 1339, also known as Tyloxapol. After thirty minutes, the animals were intraperitoneally inoculated with 50 mg/kg of each of the test compounds. Seven animals were treated with the vehicle alone and were considered the control group. A second group of six mice received 50 mg/Kg of simvastatin. Finally, a third group of seven mice received 50 mg/Kg of NST0037. Twenty-four hours after the treatments, blood was again extracted from the fasting animals and the plasma was obtained from said blood. Figure 18 shows the number of times the total cholesterol increases twenty-four hours after the administration of Triton 1339 followed by the administration of the vehicle, simvastatin or NST0037 in the different groups of mice, demonstrating that both test compounds have a hypocholesterolemic effect, being statistically significant only in the case of treatment with NST0037. In relation to the different cholesterol fractions, both compounds reduced the low density lipoprotein cholesterol (LDL-c) levels, said reduction being statistically significant only in the case of NST0037. Only simvastatin reduced the very low density lipoprotein cholesterol (VLDL-c) levels, the high density lipoprotein cholesterol (HDL-c) levels not being changed with either of the two compounds, as shown in Figures 19 to 21. Unlike simvastatin, NST0037 reduced the esterified cholesterol (EC) levels, as shown in Figure 22. In relation to the free cholesterol (FC) levels, both compounds reduced the levels of this fraction in a similar manner, as shown in Figure 23.

The results shown in this section surprisingly demonstrate that compound NST0037 has a hypocholesterolemic effect identical to or greater than simvastatin, reducing TC and LDL-c levels in a statistically significant manner. These results confirm the hypocholesterolemic effect of NST0037 observed in the apoB100 mice model.

### EXAMPLE 7

### Biosafety of NST0037 in zebrafish embryo

### 7.1. Analysis of the survival rate of compound NST0037 in comparison with simvastatin

To evaluate the biosafety of compound NST0037, its toxicological effects on the zebrafish embryo model were analyzed by means of determining the safety parameters of the OECD C15 protocol.

The fertilized eggs were obtained by natural mating of the zebrafish (*Danio rerio,* AB strain). A total of 8-10 pairs were used for each cross and a total of 200-250 eggs were generated on average per pair. The eggs were collected immediately after spawning and were washed with dilution water (CaCl₂•2H₂O₂ 0.29 g/L, MgSO₄•7H₂O₂ 0.12 g/L, NaHCO₃ 0.065 g/L, KCl 0.006 g/L), the pH being adjusted to 7.8 ± 0.2, in accordance with EC Regulation 440/2008, method C.1, and were deposited in a Petri dish.

To assure exposure in the earliest stages of development, the eggs were quickly transferred (a minimum of 50 per condition) to another Petri dish with solutions of the compounds to be tested. Subsequently, only the fertilized eggs (10 per experimental condition) were transferred from the Petri dishes to the exposure chambers (M24 microtiter plates) by means of pipettes and exposed to the substances to be tested (NST0037 or simvastatin) with dilution water (controls) or with different concentrations of the treatments. The embryos were incubated without additional aeration, at the suitable temperature (25 ± 1°C) and under a regimen of 12 hours light/12 hours darkness. Each experiment was performed in triplicate.

The studies were conducted in a total of 9 days post-fertilization, in which the treatment was renewed every day, thus conducting a semi-static assay. The treatments conducted followed the following regimen:
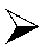 10 control animals, treated with dilution water and with daily replacement for the 9 days that the assay lasted.
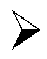 30 animals treated with compound NST0037 (10 embryos in triplicate) diluted in dilution water and with daily replacement for the 9 days that the assay lasted.
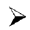 30 animals treated with the compound simvastatin (10 embryos in triplicate) diluted in dilution water and with daily replacement for the 9 days that the assay lasted.

The mortality of the embryos-larvae was recorded for the entire duration of the assay, the results being shown in Figures 24 and 25 by means of Kaplan-Meier curves. The results of the study of the mortality induced by the two substances under study showed that compound NST0037 is surprisingly safer than simvastatin at the evaluated doses, the survival curves being statistically different when comparing both treatments.

### 7.2. Analysis of the lethal dose 50s over time of compound NST0037 in comparison with simvastatin

Based on the results of the previous section, the inventors decided to investigate if the higher biosafety of compound NST0037 in comparison with simvastatin was corroborated by means of the analysis of the lethal dose 50s (LD₅₀) over time.

The fertilized eggs were obtained by natural mating of the zebrafish *(Danio rerio,* AB strain). A total of 8-10 pairs were used for each cross and a total of 200-250 eggs were generated on average per pair. The eggs were collected immediately after spawning and were washed with dilution water (CaCl₂•2H₂O₂ 0.29 g/L, MgSO₄•7H₂O₂ 0.12 g/L, NaHCO₃ 0.065 g/L, KCl 0.006 g/L) the pH being adjusted to 7.8 ± 0.2, in accordance with EC Regulation 440/2008, method C.1, and deposited in a Petri dish.

To assure exposure in the earliest stages of development, the eggs were quickly transferred (a minimum of 50 per condition) to another Petri dish with solutions of the compounds to be tested. Subsequently, only the fertilized eggs (10 per experimental condition) were transferred from the Petri dishes to the exposure chambers (M24 microtiter plates) by means of pipettes and exposed to the substances to be tested (NST0037 or simvastatin) with dilution water (controls) or with different concentrations of the treatments. The embryos were incubated without additional aeration, at the suitable temperature (25 ± 1°C) and under a regimen of 12 hours light/12 hours darkness. Each experiment was performed in triplicate.

The studies were conducted in a total of 9 days post-fertilization, in which the treatment was renewed every day, thus conducting a semi-static assay. The treatments conducted followed the following regimen:
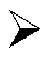 10 control animals, treated with dilution water and with daily replacement for the 9 days that the assay lasted.
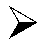 30 animals treated with compound NST0037 (10 embryos in triplicate) diluted in dilution water and with daily replacement for the 9 days that the assay lasted.
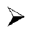 30 animals treated with the compound simvastatin (10 embryos in triplicate) diluted in dilution water and with daily replacement for the 9 days that the assay lasted.

For the duration of the study, the number of dead embryos/larvae was recorded at 24-hour intervals and the LD₅₀ was calculated at each time point of the experiment, as shown in Figure 26. The results show that in the first days posttreatment (up to day 2) the LD₅₀ of both compounds is above the maximum dose evaluated. Nevertheless, from the third day and up to the end of the experiment, simvastatin shows an LD₅₀ lower than that of compound NST0037, indicating its higher toxicity. After day 2 of treatment, the LD₅₀ of both compounds progressively reduced throughout the entire experiment, although simvastatin always showed an LD₅₀ under that of compound NST0037, indicating that NST0037 has a higher degree of biosafety than simvastatin.

### 7.3. Analysis of the percentage of healthy larvae at the end of the experiment of compound NST0037 in comparison with simvastatin over time

Based on the results of the previous sections, the inventors decided to investigate if the higher biosafety of compound NST0037 in comparison with simvastatin was corroborated by means of the analysis of the percentage of healthy larvae at the end of the experiment.

The fertilized eggs were obtained by natural mating of the zebrafish (*Danio rerio,* AB strain). A total of 8-10 pairs were used for each cross and a total of 200-250 eggs were generated on average per pair. The eggs were collected immediately after spawning and were washed with dilution water (CaCl₂•2H₂O₂ 0.29 g/L, MgSO₄•7H₂O₂ 0.12 g/L, NaHCO₃ 0.065 g/L, KCl 0.006 g/L) the pH being adjusted to 7.8 ± 0.2, in accordance with EC Regulation 440/2008, method C.1, and deposited in a Petri dish.

To assure exposure in the earliest stages of development, the eggs were quickly transferred (a minimum of 50 per condition) to another Petri dish with solutions of the compounds to be tested. Subsequently, only the fertilized eggs (10 per experimental condition) were transferred from the Petri dishes to the exposure chambers (M24 microtiter plates) by means of pipettes and exposed to the substances to be tested (NST0037 or simvastatin) with dilution water (controls) or with different concentrations of the treatments. The embryos were incubated without additional aeration, at the suitable temperature (25±1 °C) and under a regimen of 12 hours light/12 hours darkness. Each experiment was performed in triplicate.

The studies were conducted in a total of 9 days post-fertilization, in which the treatment was renewed every day, thus conducting a semi-static assay. The treatments conducted followed the following regimen:
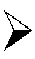 10 control animals, treated with dilution water and with daily replacement for the 9 days that the assay lasted.
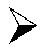 30 animals treated with compound NST0037 (10 embryos in triplicate) diluted in dilution water and with daily replacement for the 9 days that the assay lasted.
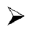 30 animals treated with the compound simvastatin (10 embryos in triplicate) diluted in dilution water and with daily replacement for the 9 days that the assay lasted.

The results at the end of the experiment allowed collecting the percentage of healthy larvae which reach the end of the experiment, defined as the number of live larvae and without symptoms of external physiopathological anomalies (morphological and/or behavioral), this being a parameter determining the biosafety of a substance under study and is complementary to the survival rates and to the LD₅₀. As shown in Figure 27, evident differences were observed between the percentages of healthy larvae between the two treatments evaluated at the higher doses under study (0.06 and 0.2 mg/L), more healthy larvae reaching the end of the experiment with the NST0037 treatment, indicating its higher biosafety.

### 7.4. Analysis of the percentage of larvae with malformations or anomalous appearance at the end of the experiment of compound NST0037 in comparison with simvastatin over time

Based on the results of the previous sections, the inventors decided to investigate if the higher biosafety of compound NST0037 in comparison with simvastatin was corroborated by means of the analysis of the percentage of larvae with malformations or anomalous appearance, collecting the number of larvae showing bodily and/or pigmentary anomalies, as well as the yolk sac reabsorption phase at suitable intervals (every 24 hours). The anomalies recorded in the study are described below:
- Intracranial thrombosis: a clot inside the blood vessel, in this case of any cerebral vessel.
- Cardiac thrombosis: occurs in a cardiac vessel.
- Cardiac edema (or hydrops): accumulation of fluid in the intercellular tissue space and also in the cavities of the organism. In the case of the heart, it causes the accumulation of fluid in the pericardial sac.
- Malformation: a noticeable difference in the shape of the body or part of the body, or organ of the body (internal or external) compared with the average shape of the part in question. The malformations observed in this study are usually of the yolk sac.

The fertilized eggs were obtained by natural mating of the zebrafish (*Danio rerio,* AB strain). A total of 8-10 pairs were used for each cross and a total of 200-250 eggs were generated on average per pair. The eggs were collected immediately after spawning and were washed with dilution water (CaCl₂•2H₂O₂ 0.29 g/L, MgSO₄•7H₂O₂ 0.12 g/L, NaHCO₃ 0.065 g/L, KCl 0.006 g/L) the pH being adjusted to 7.8 ± 0.2, in accordance with EC Regulation 440/2008, method C.1, and deposited in a Petri dish.

To assure exposure in the earliest stages of development, the eggs were quickly transferred (a minimum of 50 per condition) to another Petri dish with solutions of the compounds to be tested. Subsequently, only the fertilized eggs (10 per experimental condition) were transferred from the Petri dishes to the exposure chambers (M24 microtiter plates) by means of pipettes and exposed to the substances to be tested (NST0037 or simvastatin) with dilution water (controls) or with different concentrations of the treatments. The embryos were incubated without additional aeration, at the suitable temperature (25±1 °C) and under a regimen of 12 hours light/12 hours darkness. Each experiment was performed in triplicate.

The studies were conducted in a total of 9 days post-fertilization, in which the treatment was renewed every day, thus conducting a semi-static assay. The treatments conducted followed the following regimen:
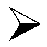 10 control animals, treated with dilution water and with daily replacement for the 9 days that the assay lasted.
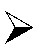 30 animals treated with compound NST0037 (10 embryos in triplicate) diluted in dilution water and with daily replacement for the 9 days that the assay lasted.
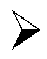 30 animals treated with the compound simvastatin (10 embryos in triplicate) diluted in dilution water and with daily replacement for the 9 days that the assay lasted.

The results indicate that the percentage of larvae with malformations or anomalous appearance is dependent on time and on the treatment, as shown in Figure 28, observing that at the evaluated dose (0.2 mg/L) simvastatin induced in the embryoslarvae a significant percentage of anomalies or toxicological problems, whereas compound NST0037 did not show significant toxicological effects, indicating its higher biosafety. Treatment with simvastatin caused intracranial and cardiac thrombosis and pericardial edemas. The data of the chart also shows that the animals recovered from the problems induced by simvastatin over time, indicating that the treatment with 0.2 mg/L of this substance was not toxic enough to induce the death of the animal, with the subsequent improvement of said animals.

These results indicate that compound NST0037 is safer than simvastatin, since the latter induces a significantly higher percentage of more severe toxicological problems.

### 7.5. Analysis of the variation of the cardiotoxicity of compound NST0037 in comparison with simvastatin dependent on the dose

Based on the results of the previous sections, the inventors decided to investigate if the higher biosafety of compound NST0037 in comparison with simvastatin was corroborated by means of the analysis of the cardiotoxicity after the treatment with the different compounds and at various doses. The study of this parameter (cardiotoxicity) not only determines the cardiac rhythm of the animals but it furthermore allows observing heartbeat alterations (tachycardia, bradycardia, etc) or heart development anomalies (pericarditis, atrophy, hypertrophy, etc.).

The fertilized eggs were obtained by natural mating of the zebrafish (*Danio rerio,* AB strain). A total of 8-10 pairs were used for each cross and a total of 200-250 eggs were generated on average per pair. The eggs were collected immediately after spawning and were washed with dilution water (CaCl₂•2H₂O₂ 0.29 g/L, MgSO₄•7H₂O₂ 0.12 g/L, NaHCO₃ 0.065 g/L, KCl 0.006 g/L) the pH being adjusted to 7.8 ± 0.2, in accordance with EC Regulation 440/2008, method C.1, and deposited in a Petri dish.

To assure exposure in the earliest stages of development, the eggs were quickly transferred (a minimum of 50 per condition) to another Petri dish with solutions of the compounds to be tested. Subsequently, only the fertilized eggs (10 per experimental condition) were transferred from the Petri dishes to the exposure chambers (M24 microtiter plates) by means of pipettes and exposed to the substances to be tested (NST0037 or simvastatin) with dilution water (controls) or with different concentrations of the treatments. The embryos were incubated without additional aeration, at the suitable temperature (25±1 °C) and under a regimen of 12 hours light/12 hours darkness. Each experiment was performed in triplicate.

The studies were conducted in a total of 9 days post-fertilization, in which the treatment was renewed every day, thus conducting a semi-static assay. The treatments conducted followed the following regimen:
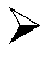 10 control animals, treated with dilution water and with daily replacement for the 9 days that the assay lasted.
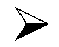 30 animals treated with compound NST0037 (10 embryos in triplicate) diluted in dilution water and with daily replacement for the 9 days that the assay lasted.
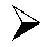 30 animals treated with the compound simvastatin (10 embryos in triplicate) diluted in dilution water and with daily replacement for the 9 days that the assay lasted.

The cardiac rhythm of the zebrafish embryos-larva was determined by means of visual analysis in stereomicroscopy, determining the heartbeat with the aid of a manual counter for a period of one minute. The different treatments varied the cardiac rhythm of the animals, as shown in Figure 29, observing that the larvae treated with simvastatin showed a statistically significant reduction with respect to the controls after the dose of 0.06, which became very evident at the dose of 2 mg/L. In contrast, the reduction of the cardiac rhythm with compound NST0037 was statistically significant only at the dose of 2 mg/L in comparison with the controls, whereas at lower concentrations it showed no differences with the controls. Furthermore, at the maximum dose used of both compounds (2 mg/L), the cardiac rhythm of the animals treated with simvastatin was statistically lower than those treated with NST0037, demonstrating again that compound NST0037 has a higher biosafety than simvastatin. Therefore, NST0037 is a more heart-safe compound than simvastatin.

### EXAMPLE 8

### Fungicidal activity of NST0037

The fungicidal activity of NST0037 was analyzed by means of bioassay against *Candida albicans.* Solutions of the β-hydroxy acid form of NST0037, lovastatin, atorvastatin and simvastatin were prepared for this purpose. The assayed concentrations were the following: 2, 1.5, 1, 0.5, 0.25, 0.125, 0.06, 0.025 and 0.01 mM. Plates of MA medium (containing per liter: 20 g malt extract, 20 g glucose, 1 g mycopeptone and 10 g agar) previously inoculated with a culture of *Candida albicans* CECT (Spanish Type-Culture Collection) 1002 were prepared. With aid of a die (6 mm in diameter) various wells were prepared in which 40 µL of the previous solutions were deposited. The study was performed in triplicate and triplicates for each compound and concentration were included in each plate. The plates were kept at 4°C for 1 hour and were subsequently incubated at 28°C overnight. The presence of antifungal activity was determined by means of the formation of *C. albicans* growth inhibition halos.

The obtained results showed that compound NST0037 has a higher antifungal activity than the statins included in the assay. As shown in Figure 30, this behavior was observed for the entire range of concentrations. In the case of higher concentrations (2-0.25 mM) compound NST0037 showed values slightly greater than those of simvastatin, the statin which showed the highest fungicidal activity. However, when using lower concentrations (0.06 and 0.025 mM), compound NST0037 was the only one capable of causing inhibition halos.

### EXAMPLE 9

### Induction of the cell expression of the 24-dehydrocholesterol reductase gene (Seladin-1/DHCR24) due to treatment with NST0037

The assay was performed on human neuroblastoma SK-N-MC cells in culture from the American Type Culture Collection (ATCC), in all cases strict rules of sterility were followed and the manipulation was performed in class II biological safety cabinets following European standard EN 12469. The cells were maintained in the following culture medium: Minimum Essential Medium Eagle (MEM) supplemented with 1 mM sodium pyruvate, 2 mM L-glutamine, 0.1 mM non-essential amino acids, 0.05 mg/ml gentamicin and 10% fetal bovine serum.

The *Whole Human Genome* microarray (Agilent Technologies) was used, which recognizes 41,000 sequences of the human genome, to analyze the gene expression profile of a culture of SK-N-MC cells treated for 24 hours in the absence (control) or presence of NST0037 at 0.4 µM or NST0037 at 4 µM. The total RNA was extracted by means of the *High Pure RNA Isolation* kit (Roche) and the amount and quality of the RNA were analyzed by means of spectrophotometry in the NanoDrop 1000 (Thermo Scientific). The manufacturer's reagents were used and the manufacturer's labeling, hybridization and washing protocols were followed (Agilent Technologies). The images obtained were processed and the fluorescence data quantified by means of Software Feature Extraction vs. 9.5 (Agilent Tecnologies). A total of 4 biological replicas of each condition were analyzed.

The results indicated, as shown in Table 1, that the seladin-1/DHCR24 gene (NCBI Reference Sequence: NM_014762.3) increased its expression with treatment with NST0037 both at 0.4 µM (1.35 times with respect to the control) and with 4 µM (1.67-fold), this last result being statistically significant (FDR < 0.05).

These results were confirmed by means of quantitative RT-PCR. The SK-N-MC cells were treated for 24 hours in the absence (control) or presence of NST0037 to 0.4 µM or NST0037 to 4 µM. The total RNA was extracted by means of the *High Pure RNA Isolation* kit (Roche) and the amount and quality of the RNA were analyzed by means of spectrophotometry in the NanoDrop 1000 (Thermo Scientific). The RT-PCR was performed by means of two steps, first the mRNA was transformed into cDNA using the *RNA to cDNA kit* (Applied Biosystem) and gene expression was subsequently analyzed by means of TaqMan probes using the validated probes Hs00207388_m1 for seladin-1/DHCR24 and Hs99999901_s1 for 18S (used to normalize the results) in the 7500 Fast Real-Time PCR System equipment (Applied Biosystem). Two independent assays were conducted in triplicate. The relative amount of expression of the gene was determined using the ΔΔCt method with SDS v2.1.1 software (Applied Biosystem); the expression of 18S was used as the normalizer.

The obtained results, as can be seen in Figure 31, show the increase of seladin-1/DHCR24 gene expression with the treatment with NST0037 at the two concentrations assayed. This data validates the results of the microarray, confirming that the treatment with NST0037 causes the increase of seladin-1/DHCR24 gene expression in relation to cholesterol biosynthesis and with anti-apoptotic capacity.

**Table 1: Analysis of seladin-1/DHCR24 gene expression in a DNA microarray assay after treatment with NST0037. The table shows the identification of the microarray probe (Probe ID), the accession number to the GenBank database, the official symbol of the gene in the Entrez NCBI database, the assigned biological process according to the Gene Ontology (GO) database, the fold change values of each treatment vs. control (untreated cells) and the statistical significance (FDR or expected error rate), for four independent replicas of the treatments for 24 hours with NST0037 at 0.4 µM and 4 µM.**

| **Probe ID** | **GenBank Accession No.** | **Symbol** | **GO_Biological_process** | **[NST 0037] µM** | **FOLD** | **FDr** |
|---|---|---|---|---|---|---|
| P379475 | NM_014762 | DHCR24 | GO:0006695 Cholesterol biosynthetic process | **0.4** | **1.35** | **0.5** |
| | | | GO:0006916 Apoptosis inhibitor activity | **4** | **1.67** | **0.007** |

## Claims

1. A compound of formula (I) 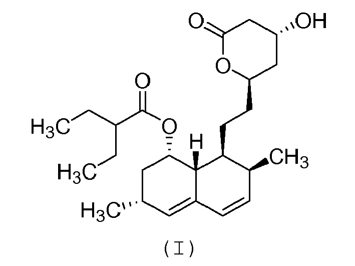 its hydroxy acid form, the pharmaceutically acceptable salts of said hydroxy acid and pharmaceutically acceptable prodrugs and solvates of the compound and of its hydroxy acid form.

2. A pharmaceutical composition comprising a compound of formula (I) according to claim 1 and/or its hydroxy acid form and/or a pharmaceutically acceptable salt of said hydroxy acid and/or a pharmaceutically acceptable prodrug or solvate of the compound or of its hydroxy acid form, and at least one pharmaceutically acceptable adjuvant, carrier and/or vehicle.

3. A compound of formula (I) according to claim 1, its hydroxy acid form or a pharmaceutically acceptable salt of said hydroxy acid and/or a pharmaceutically acceptable prodrug or solvate of the compound or of its hydroxy acid form for its use as a medicament.

4. A compound of formula (I) according to claim 1, its hydroxy acid form or a pharmaceutically acceptable salt of said hydroxy acid and/or a pharmaceutically acceptable prodrug or solvate of the compound or of its hydroxy acid form for its use in the prevention and/or the treatment of:
a. neurodegenerative diseases,
b. cognitive deterioration,
c. diseases associated with undesired oxidation,
d. age-associated pathological processes and progeria,
e. epilepsy, epileptic seizures and convulsions,
f. cardiovascular diseases such as atherosclerosis, atrial fibrillation, dyslipemia, hypercholesterolemia, hyperlipidemia, and hypertriglyceridemia, or
g. fungal or viral infections.

5. A compound of formula (I), its hydroxy acid form or a pharmaceutically acceptable salt of said hydroxy acid and/or a pharmaceutically acceptable prodrug or solvate of the compound or of its hydroxy acid form according to claim 4, wherein the neurodegenerative diseases are: Alzheimer's disease, Huntington's disease, Parkinson's disease, amyotrophic lateral sclerosis (ALS) or multiple sclerosis.

6. Use of a compound of formula (I) according to claim 1, of its hydroxy acid form, of a pharmaceutically acceptable salt of said hydroxy acid and/or of a pharmaceutically acceptable prodrug or solvate of the compound or of its hydroxy acid form in the manufacture of a medicament.

7. Use according to claim 6, wherein the medicament is used in the prevention and/or the treatment of:
a. neurodegenerative diseases,
b. cognitive deterioration,
c. diseases associated with undesired oxidation,
d. age-associated pathological processes and progeria,
e. epilepsy, epileptic seizures and convulsions,
f. cardiovascular diseases such as atherosclerosis, atrial fibrillation, dyslipemia, hypercholesterolemia, hyperlipidemia, and hypertriglyceridemia, or
g. fungal or viral infections.

8. A compound of formula (I) according to claim 1, its hydroxy acid form or a pharmaceutically acceptable salt of said hydroxy acid and/or a pharmaceutically acceptable prodrug or solvate of the compound or of its hydroxy acid form for its use in increasing seladin-1/DHCR24 gene expression.

9. A compound of formula (I) according to claim 1, its hydroxy acid form or a pharmaceutically acceptable salt of said hydroxy acid and/or a pharmaceutically acceptable prodrug or solvate of the compound or of its hydroxy acid form for its use in the prevention and/or treatment of diseases related to the seladin-1/DHCR24 gene.

10. A compound of formula (I), its hydroxy acid form or a pharmaceutically acceptable salt of said hydroxy acid and/or a pharmaceutically acceptable prodrug or solvate of the compound or of its hydroxy acid form according to claim 9, wherein neuronal death associated to neurodegenerative diseases, diseases associated with undesired oxidation or age-associated pathological processes are prevented and/or treated.

11. Use of a compound of formula (I) according to claim 1, of its hydroxy acid form, of a pharmaceutically acceptable salt of said hydroxy acid and/or of a pharmaceutically acceptable prodrug or solvate of the compound or of its hydroxy acid form in the manufacture of a medicament **characterized by** increasing seladin-1/DHCR24 gene expression.

12. Use of a compound of formula (I) according to claim 1, of its hydroxy acid form, of a pharmaceutically acceptable salt of said hydroxy acid and/or of a pharmaceutically acceptable prodrug or solvate of the compound or of its hydroxy acid form in the manufacture of a medicament for the prevention and/or treatment of diseases related to the seladin-1/DHCR24 gene.
